# EUROPEAN PATENT APPLICATION

(11) **EP 4 556 605 A1**
(43) Date of publication of application: **21.05.2025**
(21) Application number: 23914612.9
(22) Date of filing: 29.12.2023
(51) Int. Cl.: C40B 50/06

(54) **DNA METHYLATION LIBRARY CONSTRUCTION METHOD AND LIBRARY OBTAINED WITH SAME, DNA HYBRIDIZATION CAPTURE METHOD AND KIT**

(30) Priority: 03.01.2023 CN 202310003907
(71) Applicant: Boe Technology Group Co., Ltd., Beijing 100015 (CN); Beijing BOE Technology Development Co., Ltd., Beijing 100176 (CN)
(72) Inventor: YE, Bangquan, Beijing 100176 (CN)
(74) Representative: Durán-Corretjer, S.L.P.
(86) International application number: PCT/CN2023/143513
(87) International publication number: WO 2024/146481

(57) **Abstract**

A DNA methylation library construction method and a library obtained with same, a DNA hybridization capture method and a kit. The construction method comprises: providing a test sample comprising a plurality of double-stranded DNA fragments; degenerating the double-stranded DNA fragments into single-stranded DNA; carrying out ligation reaction on the single-stranded DNA and a double-stranded linker sequence to obtain single-stranded DNA linked with the double-stranded linker sequence; carrying out extension reaction on the single-stranded DNA linked with the double-stranded linker sequence to form an extended fragment, the extended fragment being linked with the double-stranded linker sequence of the single-stranded DNA; carrying out oxidation reaction on cytosine having methylation modification in double-stranded DNA linked with the double-stranded linker sequence to obtain double-stranded DNA having protected cytosine; removing the extended fragment in the double-stranded DNA having the protected cytosine to obtain single-stranded DNA having protected cytosine; carrying out deamination reaction on cytosine having no methylation modification in the single-stranded DNA having the protected cytosine to obtain single-stranded DNA having uracil; and carrying out amplification reaction on the single-stranded DNA having uracil to obtain a DNA methylation library.

## Description

The present disclosure claims the priority of the Chinese Invention Patent Application No. 202310003907.2, filed to the China National Intellectual Property Administration on January 3, 2023 and entitled "Methods for Constructing a Free DNA Methylation Library and the Library, Use and Kit Thereof", the content of which is incorporated herein by reference.

### Technical Field

An embodiment of the present disclosure relates to but is not limited to the technical field of gene sequencing, and particularly to a method for constructing a free DNA methylation library and the library, use and kit thereof.

### Background

Methylation of CpG site in genomic DNA is an important epigenetic regulatory mechanism for gene expression, tissue differentiation, organ development, senescence and tumorigenesis. DNA methylation refers to that an original hydrogen atom on a fifth carbon atom of the pyrimidine ring of a cytosine base is replaced with a methyl group, forming 5-methylcytosine. Free DNA, Circulating free DNA (cfDNA), or Cell free DNA (cfDNA) is degraded DNA fragments released into plasma and has a size of about 185 bp to 200 bp. A study has shown that changes in methylation can be detected in cfDNA up to four years before clinical cancer diagnosis. In addition, nearly 30 million methylation sites are distributed across human genome, providing rich signals for cancer detection. DNA methylation occurs early in tumorigenesis, sometimes even before gene mutations. Therefore cfDNA sequencing based on epigenetic alterations (methylation) is considered a promising method for early detection of cancer.

### Summary

The following is a summary of subject matters described herein in detail. The summary is not intended to limit the protection scope of the present disclosure.

An embodiment of the present disclosure provides a method for constructing a DNA methylation library, the method for constructing comprising:
providing a test sample containing a plurality of double-stranded DNA fragments;
denaturing the double-stranded DNA fragments into single-stranded DNA;
performing a ligation reaction on the single-stranded DNA and a double-stranded adapter sequence to obtain a single-stranded DNA connected with the double-stranded adapter sequence;
performing an extension reaction on the single-stranded DNA connected with the double-stranded adapter sequence, the extension reaction forming an extended fragment, the extended fragment being connected to the double-stranded adapter sequence of the single-stranded DNA connected with the double-stranded adapter sequence to form a double-stranded DNA connected with the double-stranded adapter sequence;
performing an oxidation reaction on cytosine carrying methylation modification in the double-stranded DNA connected with the double-stranded adapter sequence to form a protected cytosine and obtain a double-stranded DNA carrying the protected cytosine;
removing the extended fragment from the double-stranded DNA carrying the protected cytosine to obtain a single-stranded DNA carrying the protected cytosine;
performing a deamination reaction on an unmethylated cytosine in the single-stranded DNA carrying the protected cytosine to form an uracil and obtain a single-stranded DNA carrying the uracil;
performing an amplification reaction on the single-stranded DNA carrying the uracil to obtain a DNA methylation library.

In an exemplary embodiment of the present disclosure, the single-stranded DNA is modified prior to ligation with the double-stranded adapter sequence, which modification doesn't include end repair, and/or end A tailing.

In an exemplary embodiment of the present disclosure, the modification includes phosphorylation.

In an exemplary embodiment of the present disclosure, the extended fragment includes at least one uracil, and the extension reaction includes realizing the extension reaction with an extension reagent including dNTP and dUTP, wherein the dNTP doesn't include dTTP.

In an exemplary embodiment of the present disclosure, a molar ratio of dNTP to dUTP is 0.5:1 to 2:1.

In an exemplary embodiment of the present disclosure, a reaction condition of the extension reaction may include: a reaction temperature of 20 °C, and a reaction time of 15 min; the reactor is maintained at a temperature of 4 °C after the extension reaction is completed.

In an exemplary embodiment of the present disclosure, the removing the extended fragment from the double-stranded DNA carrying the protected cytosine may include:
performing a digestion reaction employing an UDG enzyme to degrade the uracil in the extended fragment, thereby removing the extended fragment from the double-stranded DNA carrying the protected cytosine.

In an exemplary embodiment of the present disclosure, the digestion reaction may include a first digestion stage and a second digestion stage; the first digestion stage may be at a reaction temperature of 37 °C, and for a reaction time of 20 min; the second digestion stage may be at a reaction temperature of 50 °C, and for a reaction time of 5 min.

In an exemplary embodiment of the present disclosure, the performing a ligation reaction on the single-stranded DNA and a double-stranded adapter sequence, and the performing an extension reaction on the single-stranded DNA connected with the double-stranded adapter sequence can form a double-stranded DNA connected with the double-stranded adapter sequence; including:
connecting double-stranded adapter sequences to both ends of single-stranded DNA respectively to amplify and generate double-stranded DNA containing paired-end adapters; and/or
connecting a first double-stranded adapter sequence to a first end of the single-stranded DNA to amplify and generate a double-stranded DNA containing a single-ended adapter, and connecting a second double-stranded adapter sequence to a second end of the single-stranded DNA to generate a double-stranded DNA containing a paired-end adapter.

In an exemplary embodiment of the present disclosure, the double-stranded adapter sequence includes cytosines, and all of the cytosines are methylated. In an exemplary embodiment of the present disclosure, the double-stranded adapter sequence includes a first double-stranded adapter sequence which is a splint sequence formed by a first oligonucleotide sequence and a second oligonucleotide sequence, and a second double-stranded adapter sequence which is a splint sequence formed by a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than that of the third oligonucleotide sequence;
overhangs of the second oligonucleotide sequence and the fourth oligonucleotide sequence can be complementary to and paired with the single-stranded DNA.

In an exemplary embodiment of the present disclosure, the double-stranded adapter sequence includes a label nucleotide, wherein, the label nucleotide includes one or more of molecular barcode sequence, unique molecular identifier (UMI), sample-specific index sequence, universal primer site, and sequencing oligonucleotide for clustering and/or sequencing; and/or
partial sequences of the above label nucleotide.

In an exemplary embodiment of the present disclosure, the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4.

In an exemplary embodiment of the present disclosure, the ends of the first oligonucleotide sequence, the second oligonucleotide sequence, the third oligonucleotide sequence and the fourth oligonucleotide sequence carry or do not carry, at the ends thereof, a modification group selected from any one or more of the phosphate group Pho, amino-substituted methylene, dideoxycytosine nucleoside and C3/C6 Spacer; wherein, the methylene in the amino-substituted methylene has 6 to 12 carbon atoms, and C3, C6 in the C3/C6 Spacer represents that the methylene in the end scaffold of the Spacer and the oligonucleotide sequence has 3 or 6 carbon atoms.

In an exemplary embodiment of the present disclosure, the method for constructing may also include a purification process, the purification process including any one or more of:
after the extension reaction and before the oxidation reaction, capturing the product obtained by the extension reaction with magnetic beads, and washing the product of the extension reaction captured by the magnetic beads with ethanol and water;
after removing the extended fragment from the double-stranded DNA carrying the protected cytosine and before the deamination reaction, capturing the product obtained after removing the extended fragment with magnetic beads, and washing the product obtained after removing the extended fragment and captured by the magnetic beads with ethanol and water;
after the deamination reaction and before the amplification reaction, capturing the product obtained by the deamination reaction with magnetic beads, and washing the product of the deamination reaction captured by the magnetic beads with ethanol and water.

In an exemplary embodiment of the present disclosure, the method for constructing may further include:
hybridization reaction;
eluting and amplifying the products of the hybridization reaction;
sequencing the amplified products.

In an exemplary embodiment of the present disclosure, the hybridization reaction includes hybridization reaction employing a hybridization reagent including betaine, dimethyl sulfoxide, and a single strand binding protein.

In an exemplary embodiment of the present disclosure, the eluting includes adding a boosting agent including tetramethyl ammonium chloride and formamide.

In an exemplary embodiment of the present disclosure, the tetramethyl ammonium chloride is at a concentration of from 0.5 M to 1.5 M.

In an exemplary embodiment of the present disclosure, the double-stranded DNA fragments are cell-free DNA fragments and/or genomic DNA fragments.

In an exemplary embodiment of the present disclosure, the test sample is from whole blood, blood components, plasma, serum, urine, stool, saliva, tissue biopsy, pleural fluid, pericardial fluid, cerebrospinal fluid, or peritoneal fluid.

In an exemplary embodiment of the present disclosure, the method for constructing may also include being used in combination with other omics.

An embodiment of the present disclosure also provides uses of the method for constructing in sequencing, detecting the presence or absence of cancer, determining cancer status, monitoring cancer progression, and/or determining cancer classification.

An embodiment of the present disclosure also provides a free DNA methylation library, which is obtained by the method for constructing the DNA methylation library provided above in embodiments of the present disclosure.

An embodiment of the present disclosure also provides a method for DNA hybridization and capture, the method for DNA hybridization and capture including:
hybridization reaction;
eluting and amplifying the products of the hybridization reaction;
sequencing the amplified products.

In an exemplary embodiment of the present disclosure, the hybridization reaction includes hybridization reaction employing a hybridization reagent including betaine, dimethyl sulfoxide, and a single strand binding protein.

In an exemplary embodiment of the present disclosure, the eluting includes adding a boosting agent including tetramethyl ammonium chloride and formamide.

In an exemplary embodiment of the present disclosure, the tetramethyl ammonium chloride is at a concentration of from 0.5 M to 1.5 M.

An embodiment of the present disclosure also provides a kit for constructing a DNA methylation library, the kit is used for performing the method for constructing the DNA methylation library provided above in embodiments of the present disclosure.

In an exemplary embodiment of the present disclosure, the double-stranded adapter sequence includes a first double-stranded adapter sequence which is a splint sequence formed by a first oligonucleotide sequence and a second oligonucleotide sequence, and a second double-stranded adapter sequence which is a splint sequence formed by a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than that of the third oligonucleotide sequence;
overhangs of the second oligonucleotide sequence and the fourth oligonucleotide sequence can be complementary to and paired with the single-stranded DNA.

In an exemplary embodiment of the present disclosure, the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4.

In an exemplary embodiment of the present disclosure, the kit further includes an extension reagent used for the extension reaction, the extension reagent may include: dNTP and dUTP, wherein the dNTP doesn't include dTTP, and a molar ratio of dNTP to dUTP is 0.5:1 to 2:1.

An embodiment of the present disclosure also provides a kit for hybridization and capture, which is used for performing the method for DNA hybridization and capture described in the embodiment of the present disclosure.

In an exemplary embodiment of the present disclosure, the kit for hybridization and capture includes: a hybridization reagent employed for the hybridization reaction and a boosting agent employed for the eluting;
the hybridization reagent includes betaine, dimethyl sulfoxide and single strand binding protein; and/or,
the eluting includes adding a boosting agent including tetramethyl ammonium chloride and formamide.

In an exemplary embodiment of the present disclosure, the tetramethyl ammonium chloride is at a concentration of from 0.5 M to 1.5 M.

Other features and advantages of the present disclosure will be set forth in the following specification, and moreover, partially become more apparent from the specification, or are understood by implementing the present disclosure. Other advantages of the present disclosure may be achieved and obtained through solutions described in the specification and drawings.

### Brief Description of Drawings

Accompany drawings are used to provide understanding of technical solution of the present disclosure, and form a part of the specification. The accompany drawings and embodiments of the present disclosure are adopted to explain the technical solution of the present disclosure, and do not form limitations on the technical solution of the present disclosure.
FIG. 1 is a schematic flowchart of a method for constructing a free DNA methylation library according to an exemplary embodiment of the present disclosure;
FIG. 2A is a schematic diagram of a structure of a first adapter sequence according to an exemplary embodiment of the present disclosure;
FIG. 2B is a schematic diagram of a structure of a second adapter sequence according to an exemplary embodiment of the present disclosure;
FIG. 3A is a schematic diagram of a structure of another first adapter sequence according to an exemplary embodiment of the present disclosure;
FIG. 3B is a schematic diagram of a structure of another second adapter sequence according to an exemplary embodiment of the present disclosure;
FIG. 4A is a schematic diagram of a structure of yet a first adapter sequence according to an exemplary embodiment of the present disclosure;
FIG. 4B is a schematic diagram of a structure of yet a second adapter sequence according to an exemplary embodiment of the present disclosure;
FIG. 5A is a schematic diagram of a structure of yet another first adapter sequence according to an exemplary embodiment of the present disclosure;
FIG. 5B is a schematic diagram of a structure of yet another second adapter sequence according to an exemplary embodiment of the present disclosure;
FIG. 6 is a schematic flowchart of a method for constructing enzyme conversion double-stranded library of Comparative Examples of the present disclosure;
FIG. 7 is a comparison diagram of methylation rate results of bases 1-150 of Read2 sequence in library sequencing according to Example 1 and Comparative Examples 1-2 of the present disclosure;
FIG. 8 is a schematic flowchart of a method for DNA hybridization and capture according to an exemplary embodiment of the present disclosure;
FIG. 9 is a comparison diagram of specificity and homogeneity of target gene capture in the methods for capture according to Example 5 and Comparative Example 3 of the present disclosure.

### Detailed Description

To make the objectives, technical solutions, and advantages of the present disclosure clearer, the embodiments of the present disclosure will be described in detail below with reference to the accompany drawings. It is to be noted that the embodiments and features in the embodiments of the present disclosure may be randomly combined with each other if there is no conflict.

Implementations herein may be implemented in a plurality of different forms. Those of ordinary skills in the art can readily appreciate a fact that the implementations and contents may be varied into various forms without departing from the spirit and scope of the present disclosure. Therefore, the present disclosure should not be explained as being limited to the contents recorded in the following implementations only. The embodiments and features in the embodiments of the present disclosure may be randomly combined with each other if there is no conflict.

In the description of the present disclosure, ordinal numerals such as "first" and "second" are set to avoid confusion of constituents, but not intended for restriction in quantity.

As used herein, the term "amplicon" refers to a product of polynucleotide amplification reaction; that is, a clonal population of polynucleotide, which can be single-stranded or double-stranded, and replicates from one or more starting sequences. One or more starting sequences may be one or more copies of the same sequence, or they may be a mixture of different sequences. Preferably, amplicons are formed from the amplification of a single starting sequence. Amplicons can be produced by a variety of amplification reactions, the products of which include replicas of one or more starting or target nucleic acids. In one aspect, amplification reactions that produce amplicons are "template driven" because base pairs of the reactants (nucleotides or oligonucleotides) have, in the template polynucleotides, the complements necessary to produce a reaction product. In one aspect, template-driven reaction is primer extension using nucleic acid polymerase or oligonucleotide ligation using nucleic acid ligase. Such reactions include, but are not limited to, polymerase chain reaction (PCR), linear polymerase reaction, nucleic acid sequence based amplification (NASBA), rolling loop amplification, etc.

In the description of the present disclosure, the term "amplification" refers to performing an amplification reaction. "Reaction solution" refers a solution containing all reactants necessary to perform a reaction, which may include, but is not limited to, a buffer that maintains a pH at a selected level during a reaction, salt, cofactor, scavenger, etc..

The term "fragment" refers to a portion of a larger polynucleotide molecule. For example, polynucleotides can be broken down or cleaved into a plurality of fragments by natural processes, such as cfDNA fragments that can occur naturally in biological samples, or by in vitro manipulation. Various methods for nucleic acid cleavage are well known in the art. These methods may be, for example, chemical or physical or enzymatic. Enzymatic cleavage may include partial degradation with deoxyribonuclease; partial depurination with acids; use of restriction enzymes; intron-encoded endonuclease; DNA-based cleavage methods, such as methods for triplex and hybrid formation, which rely on specific hybridization of nucleic acid fragments to locate cleavage reagents at specific locations within nucleic acid molecules; or other enzymes or compounds that cleave polynucleotides at known or unknown locations. A method for physical cleavage may include subjecting a polynucleotide to a high shear rate. A high shear rate can be generated by, for example, moving DNA through chambers or channels with pits or spikes, or forcing DNA samples passing through size-confined flow channels, for example, pores with cross-sectional dimensions in the micron or submicron range. Other physical methods include ultrasound and atomization. A combination of physical and chemical cleavage methods, such as cleaving by heating and ion-mediated hydrolysis, can also be used. See, e.g., Sambrook et al., "Molecular Cloning: A Laboratory Handbook," 3rd ed. Cold Spring Harbor Laboratory Press, Cold Spring Harbor, NY (2001) ("Sam brook et al"), incorporated herein by reference for all purposes. These methods can be optimized to digest nucleic acids into fragments of a selected size range.

The term "PCR" refers to Polymerase Chain Reaction (PCR), a reaction in which a specific DNA sequence is amplified in vitro by simultaneous primer extension of the DNA complementary strand. In other words, PCR is a reaction used to produce a plurality of copies or duplicates of a target nucleic acid flanked by primer binding sites, which involves repeating the following steps one or more times: (I) denaturing the target nucleic acid, (ii) annealing the primer to the primer binding site, and (iii) extending the primer by nucleic acid polymerase in the presence of nucleoside triphosphate. Typically, in a thermal cycler, a reaction is cycled at different temperatures optimized for each step. Specific temperature, duration of each step, and rate of change between steps depend on a number of factors well known to those of ordinary skill in the art. For example, in conventional PCR using Taq DNA polymerase, double-stranded target nucleic acids can be denatured at > 90 °C, primers annealed at temperatures in the range of 50 °C to 75 °C, and primers extended at temperatures in the range of 72 °C to 78 °C. The term "PCR" includes derivative forms of reactions, including but not limited to RT-PCR, real-time PCR, nested PCR, quantitative PCR, multiplex PCR, etc. One skilled in the art can discern the specific form of PCR employed from the context of the application. The reaction volume may range from a few hundred nanoliters, e.g., 200 nL, to a few hundred µL, e.g., 200 pt.

"Amplification primer" means the primer used to perform amplification. Herein, "primer" refers to natural or synthetic oligonucleotides, which can act as the starting point of nucleic acid synthesis when forming a duplex with a polynucleotide template, and extend from its 3 'end along the template, thus forming an extended duplex. Primer extension is usually performed with nucleic acid polymerases, such as DNA or RNA polymerases. A nucleotide sequence added during extension is determined by the sequence of a template polynucleotide. Usually, primers are extended by DNA polymerase. Primers are typically at a length ranging from 14 to 40 nucleotides, or from 18 to 36 nucleotides. Primers are used in various nucleic acid amplification reactions, for example, linear amplification reactions using a single primer, or polymerase chain reactions using two or more primers. Guidelines for selecting the length and sequence of primers for a particular application are well known to those of ordinary skill in the art.

The term "patient" refers to a human or non-human animal that is known to have or may have a medical condition or disorder, such as a tumor.

The term "cfDNA" refers to a fragment of nucleic acid circulating in a subject (e.g., in the bloodstream) and derived from one or more healthy cells and/or one or more cancer cells.

The term "overhang" refers to the single-stranded portion of a nucleic acid strand that carries one or more unpaired nucleotides at the end.

In one embodiment, "sample" may be a sample selected from blood, plasma, serum, urine, and saliva samples. In other embodiments, a sample is a plasma sample from a patient with cancer or a patient suspected of having cancer. Alternatively, a sample may include samples selected from whole blood, blood components, tissue biopsies, pleural fluid, pericardial fluid, cerebrospinal fluid (CSF), and peritoneal fluid. According to some embodiments, a sample includes a plurality of cell-free nucleic acids (e.g., cell-free DNA (cfDNA)) fragments from healthy cells and cancer cells. Optionally, in one embodiment, cell-free nucleic acids (e.g., cfDNA) may be extracted and/or purified from a sample prior to subsequent library preparation steps. In general, any method known in the art can be used to extract and purify cell-free nucleic acids from samples. For example, cell-free nucleic acids can be extracted and purified using one or more known commercially available protocols or kits, such as the QIAamp Circulating Nucleic Acids Kit (Qiagen) or the MagMAX Cell-Free DNA Isolation Kit (Thermo Fisher Scientific). At present, there are two main methods for constructing methylation library of cfDNA methylation sequencing on the market. One is a method for constructing single-strand library based on bisulfite transformation (such as Swift Biosciences kit Accel-NGS ^{®} Methyl-Seq DNA Library Kit), which reacts cfDNA in a high-salt, high-temperature environment for 2 hours to 5 hours, which will cause cfDNA to break and degrade, and because cfDNA itself has some gap damage, so this method will cause a large number of cfDNA fragments to be lost during the library construction process, and the lost fragments may contain methylation mutation sites, so it is difficult for this method to detect methylation mutations in cfDNA at a low-starting amount, and a detection sensitivity is greatly reduced. A second one is a method for constructing double-stranded library based on enzymatic transformation, which can avoid the problem of fragment degradation caused by bisulfite treatment of cfDNA. A general process is: performing end repair directly on double-stranded cfDNA, followed by adding an adapter sequence. All cytosines (C bases) contained in the adapter sequence have methylation modifications. In a double-stranded state, methylated cytosine is converted to carboxycytosine through actions of TET2 enzyme and others, while unmethylated C base remains unchanged. Then, the double-stranded DNA is denatured into single-stranded DNAs, and the unmethylated cytosine is deaminated using APOBEC enzyme to form uracil (U base). Finally, a methylation library is formed by amplification and enrichment. Since cfDNA has a sticky end, and a length of single-stranded DNA of overhang is relatively long, one of raw materials used for end repair in this method is deoxycytidine triphosphate (dCTP), and none of carbon atoms in dCTP is methylated, and a problem of low terminal methylation often occurs after the sequencing is completed. In addition, due to a damage gap problem in the cfDNA strand itself, after an adapter ligation step, DNA strand denaturation will form a single strand of DNA with a single end adapter, which cannot be amplified in subsequent steps, resulting in the loss of DNA strands.

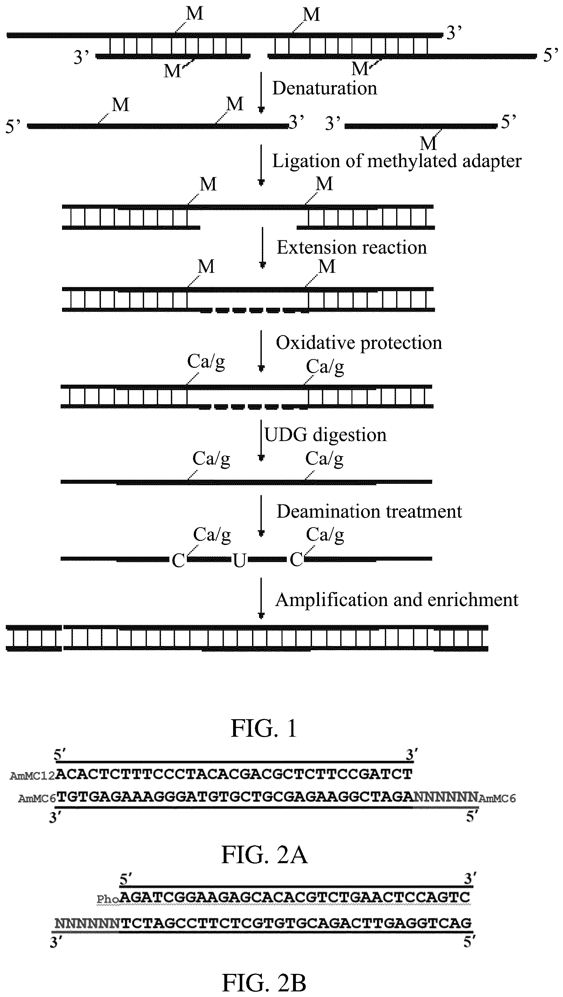

An embodiment of the present disclosure provides a method for constructing a free DNA methylation library. FIG. 1 is a schematic flowchart of a method for constructing a free DNA methylation library according to an exemplary embodiment of the present disclosure. As shown in FIG. 1, the method for constructing includes:
providing a free DNA sample;
denaturing the free DNA sample into single-stranded DNA;
performing a ligation reaction on the single-stranded DNA and an adapter sequence containing cytosine and each of the cytosine having methylation modification, so that both ends of the single-stranded DNA each being connected with one adapter sequence, to obtain a single-stranded DNA having an adapter sequence at both ends;
performing an extension reaction on the single-stranded DNA connected with an adapter sequence at both ends, the extension reaction forming an extended fragment, and the extended fragment being respectively connected at both ends with the adapter sequence of the single-stranded DNA which is connected with an adapter sequence at both ends, to form a double-stranded DNA connected with an adapter sequence at both ends;
performing an oxidation reaction on cytosine carrying methylation modification in the double-stranded DNA to form a protected cytosine and obtain a double-stranded DNA carrying the protected cytosine;
removing the extended fragment from the double-stranded DNA carrying the protected cytosine to obtain single-stranded DNA carrying the protected cytosine;
performing a deamination reaction on an unmethylated cytosine in the single-stranded DNA carrying the protected cytosine to form an uracil and obtain a single-stranded DNA carrying the uracil;
performing an amplification reaction on the single-stranded DNA carrying the uracil to obtain a free DNA methylation library.

The method for constructing a free DNA methylation library provided by the embodiment of the present disclosure does not need to use bisulfite to perform methylation conversion treatment on cfDNA, and reaction conditions are mild, which solves problems of excessive degradation and damage loss of cfDNA. Moreover, the method for constructing a free DNA methylation library provided by an embodiment of the present disclosure does not perform end repair, which solves a problem of 3'-end methylation distortion, and first denatures a double-stranded DNA sample into a single-stranded DNA, and then performs adapter ligation in a single-stranded state, thereby ensuring that the single strands formed by the denatured cfDNA strand gap are effectively utilized, and avoiding a problem of strand loss caused by traditional double-strand ligation. Therefore, the method for constructing a free DNA methylation library provided by the embodiment of the present disclosure can avoid fragment loss of free DNA, thereby can improve a yield of the methylation library, and can improve an accuracy of methylation rate detection.

In an exemplary embodiment of the present disclosure, the extended fragment includes at least one uracil.

In an exemplary embodiment of the present disclosure, the reagents employed for the extension reaction may include Klenow reaction buffer, a mixture of dNTP (not including dTTP) and dUTP, an aqueous solution of Tween 20, and Klenow Fragment.

In an exemplary embodiment of the present disclosure,
the mixture of the dNTP and dUTP may be at an operating concentration of 0.1 mM to 0.4 mM;
in the aqueous solution of Tween 20, a mass of Tween 20 to a volume of water is at a ratio of (0.03 to 0.1) g: 100 ml;
the Klenow Fragment may be at an operating concentration of from 0.02 U/µL to 0.08 U/µL.

In an exemplary embodiment of the present disclosure, a reaction condition of the extension reaction may include: a reaction temperature of 20 °C, and a reaction time of 15 min; and the reactor is maintained at a temperature of 4 °C after the extension reaction is completed.

In an exemplary embodiment of the present disclosure, removing the extended fragment from the double-stranded DNA carrying the protected cytosine may include:
performing a digestion reaction employing an UDG enzyme to degrade the uracil in the extended fragment, thereby removing the extended fragment from the double-stranded DNA carrying the protected cytosine.

In an exemplary embodiment of the present disclosure, the digestion reaction may include a first digestion stage and a second digestion stage; the first digestion stage may be at a reaction temperature of 37 °C, and for a reaction time of 20 min; the second digestion stage may be at a reaction temperature of 50 °C, and for a reaction time of 5 min.

In an exemplary embodiment of the present disclosure, the adapter sequence may include a first adapter sequence and a second adapter sequence; the first adapter sequence is formed from a first oligonucleotide sequence having at least 70% identity to a sequence set forth in SEQ ID NO. 1 and a second oligonucleotide sequence having at least 70% identity to a sequence set forth in SEQ ID NO. 2; the second adapter sequence is formed from a third oligonucleotide sequence having at least 70% identity to a sequence set forth in SEQ ID NO. 3 and a fourth oligonucleotide sequence having at least 70% identity to a sequence set forth in SEQ ID NO. 4.

In an exemplary embodiment of the present disclosure, the reagents employed for the ligation reaction may also include an aqueous solution of polyethylene glycol, DNA ligase buffer, dithiothreitol, adenine nucleoside triphosphate, polynucleotide kinase, and DNA ligase.

In an exemplary embodiment of the present disclosure,
in the aqueous solution of polyethylene glycol, a mass of polyethylene glycol to a volume of water may be at a ratio of (5 to 20) g: 100 ml;
the dithiothreitol can be at an operating concentration of 0.01 M to 0.05 M;
the adenine nucleoside triphosphate may be at an operating concentration of from 0.5 mM to 2 mM;
the polynucleotide kinase may be at an operating concentration of from 0.1 U/µL to 0.4 U/µL;
the DNA ligase may be at an operating concentration of from 2 U/µL to 5 U/µL.

In an exemplary embodiment of the present disclosure, the reaction conditions of the ligation reaction may include a reaction temperature of 37 °C and a reaction time of 45 min.

In an exemplary embodiment of the present disclosure, the protected cytosine may be carboxyl cytosine.

In an exemplary embodiment of the present disclosure, the reagents employed for the oxidation reaction may include: TET2 enzyme, TET2 reaction buffer, oxidative supplement, dithiothreitol, oxidative boosting agent, nuclease-free water, and Fe (II).

In an exemplary embodiment of the present disclosure, the reaction conditions of the oxidation reaction may include: a reaction temperature of 37 °C and a reaction time of 60 min; after the oxidation reaction is completed, a stopping solution is added and incubated at 37 °C for 30 min.

In an exemplary embodiment of the present disclosure, the reagents employed for the deamination reaction may include APOBEC protein, APOBEC reaction buffer, bovine serum protein, and nuclease-free water.

In an exemplary embodiment of the present disclosure, the reaction conditions of the deamination reaction may include a reaction temperature of 37 °C and a reaction time of 180 min.

In an exemplary embodiment of the present disclosure, reagents employed for the amplification reaction may also include Index Primer Mix and HiFi HotStart Uracil Mix.

In an exemplary embodiment of the present disclosure, the reaction conditions of the amplification reaction may include: reaction at 98 °C for 30 s; followed by 7 to 16 cycles of reaction 98 °C for 10 s, reaction at 62 °C for 30 s, and reaction at 65 °C for 60 s; reaction at 65 °C for 5 min. After the amplification reaction is completed, the reactor is maintained at a temperature of 4 °C.

In an exemplary embodiment of the present disclosure, the method for constructing may also include a purification process, which includes any one or more of:
after the extension reaction and before the oxidation reaction, capturing the product obtained by the extension reaction with magnetic beads, and washing the product of the extension reaction captured by the magnetic beads with ethanol and water;
after removing the extended fragment from the double-stranded DNA carrying the protected cytosine and before the deamination reaction, capturing the product obtained after removing the extended fragment with magnetic beads, and washing the product obtained after removing the extended fragment and captured by the magnetic beads with ethanol and water;
after the deamination reaction and before the amplification reaction, capturing the product obtained by the deamination reaction with magnetic beads, and washing the product of the deamination reaction captured by the magnetic beads with ethanol and water.

An embodiment of the present disclosure also provides a free DNA methylation library, which is obtained by the method for constructing the free DNA methylation library provided above in embodiments of the present disclosure.

An embodiment of the present disclosure also provides a method for sequencing free DNA methylation, wherein the method for sequencing includes: using the method for constructing a free DNA methylation library provided above in an embodiment of the present disclosure to construct a free DNA methylation library, and then sequencing.

An embodiment of the present disclosure also provides a kit for constructing a free DNA methylation library, which is used for performing the method for constructing the free DNA methylation library provided above in embodiments of the present disclosure.

The kit includes: adapter sequences, ligation reagent for adapter sequences, an extension reagent, an oxidative protection reagent, a reagent for removing extended fragment, a deamination reagent, and an amplification reagent.

In an exemplary embodiment of the present disclosure, the adapter sequence may include a first adapter sequence and a second adapter sequence; the first adapter sequence is formed from a first oligonucleotide sequence having at least 70% identity to a sequence set forth in SEQ ID NO. 1 and a second oligonucleotide sequence having at least 70% identity to a sequence set forth in SEQ ID NO. 2; the second adapter sequence is formed from a third oligonucleotide sequence having at least 70% identity to a sequence set forth in SEQ ID NO. 3 and a fourth oligonucleotide sequence having at least 70% identity to a sequence set forth in SEQ ID NO. 4.

In an exemplary embodiment of the present disclosure, the ligation reagent for the adapter sequence may include an aqueous solution of polyethylene glycol, DNA ligase buffer, dithiothreitol, adenine nucleoside triphosphate, polynucleotide kinase, and DNA ligase.

In an exemplary embodiment of the present disclosure, the extension reagents may include Klenow reaction buffer, a mixture of dNTP (not including dTTP) and dUTP, an aqueous solution of Tween 20, and Klenow Fragment.

In an exemplary embodiment of the present disclosure, the oxidative protection reagent may include: TET2 enzyme, TET2 reaction buffer, oxidative supplement, dithiothreitol, oxidative boosting agent, nuclease-free water, and Fe (II).

In an exemplary embodiment of the present disclosure, the reagent for removing extended fragment may include an UDG enzyme.

In an exemplary embodiment of the present disclosure, the deamination reagent may include APOBEC protein, APOBEC reaction buffer, bovine serum protein, and nuclease-free water.

In an exemplary embodiment of the present disclosure, the amplification reagent may also include Index Primer Mix and HiFi HotStart Uracil Mix.

An embodiment of the present disclosure provides a method for constructing a DNA methylation library, including.
providing a test sample containing a plurality of double-stranded DNA (dsDNA) fragments;
denaturing the double-stranded DNA fragments into single-stranded DNA;
performing a ligation reaction on the single-stranded DNA and a double-stranded adapter sequence to obtain a single-stranded DNA connected with the double-stranded adapter sequence;
performing an extension reaction on the single-stranded DNA connected with the double-stranded adapter sequence, the extension reaction forming an extended fragment, the extended fragment being connected to the double-stranded adapter sequence of the single-stranded DNA connected with the double-stranded adapter sequence, to form a double-stranded DNA connected with the double-stranded adapter sequence;
performing an oxidation reaction on cytosine carrying methylation modification in the double-stranded DNA connected with the double-stranded adapter sequence to form a protected cytosine and obtain a double-stranded DNA carrying the protected cytosine;
removing the extended fragment from the double-stranded DNA carrying the protected cytosine to obtain a single-stranded DNA carrying the protected cytosine;
performing a deamination reaction on an unmethylated cytosine in the single-stranded DNA carrying the protected cytosine to form an uracil and obtain a single-stranded DNA carrying the uracil;
performing an amplification reaction on the single-stranded DNA carrying the uracil to obtain a DNA methylation library.

At present, an adapter employed in single-stranded DNA ligation is a single-stranded oligonucleotide adapter. A ligation product formed by this way for ligation is single-stranded, with poor stability, and requires expensive single-stranded DNA ligase to realize the ligation. To a large extent, the cost of reagents will be increased. An exemplary embodiment of the present disclosure employs a double-stranded adapter to perform single-stranded DNA ligation. N base of an overhang of a long strand of the double-stranded adapter can be complementary to the single-stranded DNA to be ligated, and a short strand of a splint adapter is complementary to and paired with the long strand of the double-stranded adapter, so that the single-stranded DNA to be ligated and the double-stranded adapter present a double-stranded state at the junction of the short strand (5' end) of the double-stranded adapter. At this time, an effective ligation reaction can be completed by using a conventional and cheap double-stranded DNA ligase (such as T4 DNA Ligase), and the cost can be greatly reduced.

In an exemplary embodiment of the present disclosure, the single-stranded DNA is modified prior to ligation with a double-stranded adapter sequence, which modification doesn't include end repair, and/or end A tailing.

In an exemplary embodiment of the present disclosure, the modification includes phosphorylation.

In an exemplary embodiment of the present disclosure, denaturation of the double-stranded DNA fragment may include heating the double-stranded DNA fragment to a temperature to melt the double strands of the double-stranded DNA fragment into a single strand. The denaturation may be at a temperature of 95 °C, and for time period of 5 min.

In an exemplary embodiment of the present disclosure, the extended fragment includes at least one uracil.

In an exemplary embodiment of the present disclosure, the reagents employed for the extension reaction may include Klenow reaction buffer, a mixture of dNTP (not including dTTP) and dUTP, Tween 20, and Klenow Fragment.

In an exemplary embodiment of the present disclosure, the mixture of the dNTP and dUTP may be at an operating concentration of from 0.1 mM to 0.4 mM.

In an exemplary embodiment of the present disclosure, a molar ratio of dNTP to dUTP is 0.5:1 to 2:1.
in the aqueous solution of Tween 20, a mass of Tween 20 to a volume of water is at a ratio of (0.03 to 0.1) g: 100 ml, which can also be understood as that the aqueous solution of Tween 20 is at a mass concentration of 0.03% to 0.1%; and/or,
the Klenow Fragment may be at an operating concentration of from 0.02 U/µL to 0.08 U/µL.

In an exemplary embodiment of the present disclosure, the Klenow reaction buffer may be at an operating concentration of from 0.5 × to 2 ×.

In an exemplary embodiment of the present disclosure, a reaction condition of the extension reaction may include: a reaction temperature of 20 °C, and a reaction time of 15 min; the reactor is maintained at a temperature of 4 °C after the extension reaction is completed.

In an exemplary embodiment of the present disclosure, removing the extended fragment from the double-stranded DNA carrying the protected cytosine may include:
degrading the uracil in the extended fragment, thereby removing the extended fragment from the double-stranded DNA carrying the protected cytosine.

In method for constructing a DNA methylation library of an embodiment of the present disclosure, after the ligation reaction of the single-stranded DNA is completed, a raw material containing dUTP can be used to form an extended fragment, and the extended fragment contains randomly distributed uracil, which is beneficial to degrade the uracil in the extended fragment in a subsequent step, so as to achieve a purpose of removing the extended fragment.

In an exemplary embodiment of the present disclosure, degrading the uracil in the extended fragment may include:
performing a digestion reaction employing an UDG enzyme to degrade the uracil in the extended fragment.

In an exemplary embodiment of the present disclosure, the digestion reaction may include a first digestion stage and a second digestion stage; the first digestion stage may be at a reaction temperature of 37 °C, and for a reaction time of 20 min; the second digestion stage may be at a reaction temperature of 50 °C, and for a reaction time of 5 min.

In the digestion reaction employed an UDG enzyme, the extended fragment generated in the previous step can be digested, and a sequence from the adapter at both ends will not be digested, but those adapter complementary sequences can be removed through a subsequent denaturation process.

In an exemplary embodiment of the present disclosure, performing a ligation reaction on the single-stranded DNA and a double-stranded adapter sequence, and performing an extension reaction on the single-stranded DNA connected with the double-stranded adapter sequence to form a double-stranded DNA connected with the double-stranded adapter sequence, includes:
connecting double-stranded adapter sequences to both ends of single-stranded DNA respectively to amplify and generate double-stranded DNA containing paired-end adapters; and/or
connecting a first double-stranded adapter sequence to a first end of the single-stranded DNA to amplify and generate a double-stranded DNA containing a single-ended adapter, and connecting a second double-stranded adapter sequence at a second end of the single-stranded DNA to generate a double-stranded DNA containing a paired-end adapter.

In an exemplary embodiment of the present disclosure, the double-stranded adapter sequence includes cytosines, and all of the cytosines have methylation modification.

In an exemplary embodiment of the present disclosure, the double-stranded adapter sequence includes
a label nucleotide, wherein, the label nucleotide includes one or more of molecular barcode sequence, unique molecular identifier (UMI), sample-specific index sequence, an universal primer site, and a sequencing oligonucleotide for clustering and/or sequencing; and/or
partial sequences of the above label nucleotide.

In a description of an embodiment of the present disclosure, the term "unique molecular identifier" may be interchangeable with "sequence tag", "tag" or "barcode" and refers to an oligonucleotide attached to a polynucleotide or template molecule for identifying and/or tracking polynucleotides or templates in a reaction or a series of reactions. A sequence tag may be attached to the 3'-or 5'-terminus of a polynucleotide or template, or it may be inserted into an interior of such polynucleotide or template to form a linear conjugate, sometimes referred to herein as a "labeled polynucleotide" or a "labeled template" or the like. A sequence tag can vary widely in size and composition. A length and composition of sequence tags can vary widely, and a choice of specific length and/or composition depends on several factors, including, but not limited to, how the tag is used to generate reads, for example, by hybridization reactions or by enzymatic reactions such as sequencing; whether they are labeled, e.g. with fluorescent dyes, etc.; a number of distinguishable oligonucleotide tags required to identify a group of polynucleotides, etc., and how tags for a particular group must differ to ensure reliable identification, e.g., erroneous identification without cross-hybridization or sequencing errors. In one aspect, the sequence tags may each have a length of from about 2 to about 36 nucleotides, or from about 4 to about 30 nucleotides, or from about 8 to about 20 nucleotides, or from about 6 to about 10 nucleotides. In one aspect, sets of sequence tags are used, wherein each sequence tag in one set has an unique nucleotide sequence that differs by at least two bases from a nucleotide sequence of every other tag in the same set; in another aspect, sets of sequence tags are used, wherein a sequence of each tag in one set differs by at least three bases from a sequence of every other tag in the same set.

An embodiment of the present disclosure relates to use of unique sequence tags. Unique sequence tags according to an embodiment of the present disclosure may provide a wide variety of functions. For example, the unique sequence tag may include molecular barcode sequence, unique molecular identifier (lHVfl) sequence, or index sequence. In one embodiment, unique sequence tags (e.g., barcodes or index sequences) may be used to identify DNA sequences originating from common sources, such as sample types, tissues, patients, or individuals. According to one embodiment, a barcode or index sequence may be used for multiplex sequencing. In one embodiment, unique sequence tags, such as unique molecular identifiers (UMIs), may be used to identify unique nucleic acid sequences from mixed cfDNA samples. For example, different unique sequence tags (e.g., UMIs) can be used to distinguish between ssDNA molecules, dsDNA molecules, or damaged molecules (e.g., gapped dsDNA) contained in cfDNA samples. In another embodiment, unique sequence tags (e.g., UMIs) may be used to reduce amplification bias, which is the asymmetric amplification of different targets due to differences in nucleic acid composition (e.g., high GC content). Unique sequence tags (UMIs) can be used to distinguish nucleic acid mutations that occur during amplification. Unique sequence tags may exist in a multifunctional nucleic acid adaptor, which may include an unique sequence tag and an universal priming site. In some embodiments, a length of the unique sequence tag may be greater than about 5, 6, 7, 8, 9, 10, 11, 12, 13, 14, 15, 16, 17, or 18 nucleic acids.

In an exemplary embodiment of the present disclosure, the double-stranded adapter sequence includes a first double-stranded adapter sequence which is a splint sequence formed from a first oligonucleotide sequence and a second oligonucleotide sequence, and a second double-stranded adapter sequence which is a splint sequence formed from a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than thatof the third oligonucleotide sequence;
overhangs of the second oligonucleotide sequence and the fourth oligonucleotide sequence can be complementary to and paired with the single-stranded DNA.

In a description of the present disclosure, the term "splint sequence" is intended to refer to a sequence of oligonucleotides that, when hybridized with two or more other polynucleotides, acts as a "splint" to position the polynucleotides adjacent to each other so that they can be joined together, as shown in FIG. 1.

In an exemplary embodiment of the present disclosure, the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4.

In an exemplary embodiment of the present disclosure, ligation efficiency may be improved by adjusting the paired-end modification groups of a splint sequence.

In an exemplary embodiment of the present disclosure, the first oligonucleotide sequence, the second oligonucleotide sequence, the third oligonucleotide sequence and the fourth oligonucleotide sequence carry or do not carry, at the ends thereof, a modification group selected from any one or more of the phosphate group Pho, amino-substituted methylene, dideoxycytosine nucleoside and C3/C6 Spacer; wherein, the methylene in the amino-substituted methylene has 6 to 12 carbon atoms, and C3, C6 in the C3/C6 Spacer represents that the methylene in the end scaffold of the Spacer and the oligonucleotide sequence has 3 or 6 carbon atoms.

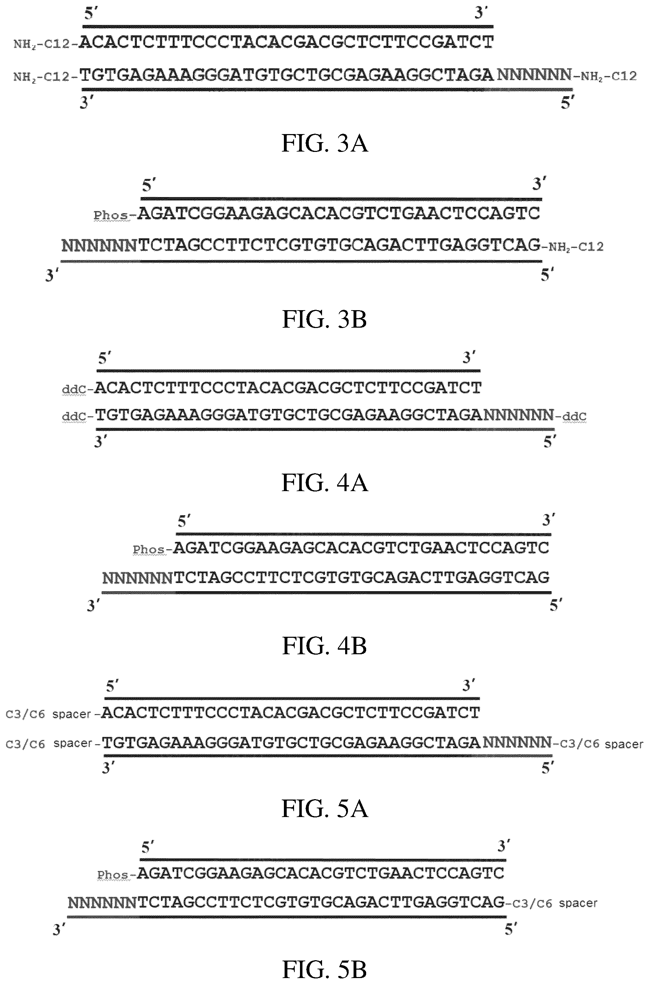

FIG. 2A is a schematic diagram of a structure of a first adapter sequence according to an exemplary embodiment of the present disclosure; FIG. 2B is a schematic diagram of a structure of a second adapter sequence according to an exemplary embodiment of the present disclosure. As shown in FIGS. 2A and 2B, Pho represents phosphate group modification, which facilitates DNA ligation; N denotes degenerate bases; AmMC6: AmM represents amino modification, and C6 represents a number of carbon atoms of a linear methylene group between an amino group and DNA, which mainly plays a role for increasing a spatial distance and preventing the adapter from self-connecting; AmMC12: AmM represents an amino modification, and C12 represents a number of carbon atoms of a linear methylene between an amino group and DNA, which mainly plays a role for increasing a spatial distance and preventing an adapter from self-connecting, and improving a ligation efficiency between an adapter and DNA fragments. FIG. 3A is a schematic diagram of a structure of another first adapter sequence according to an exemplary embodiment of the present disclosure; FIG. 3B is a schematic diagram of a structure of another second adapter sequence according to an exemplary embodiment of the present disclosure. As shown in FIGS. 3A and 3B, both the 5' end and the 3' end may employ NH₂ C12 modification, wherein C12 represents a number of carbon atoms of a linear methylene between an amino group and DNA; Pho represents phosphate group modification, which facilitates DNA ligation; N denotes degenerate bases.

FIG. 4A is a schematic diagram of a structure of yet a first adapter sequence according to an exemplary embodiment of the present disclosure; FIG. 4B is a schematic diagram of a structure of yet a second adapter sequence according to an exemplary embodiment of the present disclosure. As shown in FIGS. 4A and 4B, the 5' end and 3' end can be modified employing dideoxycytosine nucleoside (ddC). Due to a deoxidation at No. 3 carbon, ddC cannot form a phosphodiester bond with a carboxyl group of an adjacent nucleotide, so an interconnection between the adapters can be prevented, and a ligation efficiency between the adapter and the DNA fragment can be improved; Pho represents phosphate group modification, which facilitates DNA ligation; N denotes degenerate bases.

FIG. 5A is a schematic diagram of a structure of yet another first adapter sequence according to an exemplary embodiment of the present disclosure; FIG. 5B is a schematic diagram of a structure of yet another second adapter sequence according to an exemplary embodiment of the present disclosure. As shown in FIGS. 5A and 5B, the 5' end and 3' end can be modified employing C3/C6 Spacer type, C3 refers to 3 methylene CH2, and C6 refers to 6 methylene CH2, which plays a role for increasing a spatial distance at the end of the adapter, and also preventing the adapter from self-connecting; Pho represents phosphate group modification, which facilitates DNA ligation; N denotes degenerate bases.

In an exemplary embodiment of the present disclosure, the reagents employed for the ligation reaction may also include an aqueous solution of polyethylene glycol (e.g., PEG 8000), DNA ligase buffer (e.g., T4 DNA Ligase buffer), dithiothreitol (DTT), adenine nucleoside triphosphate (ATP), polynucleotide kinase (e.g., T4 PNK), and DNA ligase (e.g., T4 DNA Ligase).

In an exemplary embodiment of the present disclosure,
in the aqueous solution of polyethylene glycol, a mass of polyethylene glycol to a volume of water may be at a ratio of (5 to 20) g: 100 ml, which can also be understood as the aqueous solution of polyethylene glycol is at a mass concentration of 20 g/120 g to 5 g/105 g, about 5% to 20%;
the dithiothreitol can be at an operating concentration of 0.01 M to 0.05 M;
the adenine nucleoside triphosphate may be at an operating concentration of from 0.5 mM to 2 mM;
the polynucleotide kinase may be at an operating concentration of from 0.1 U/µL to 0.4 U/µL;
the DNA ligase may be at an operating concentration of from 2 U/µL to 5 U/µL.

In an exemplary embodiment of the present disclosure, the DNA ligase buffer may be at an operating concentration of from 0.5 × to 2 ×.

In an exemplary embodiment of the present disclosure, the reaction conditions of the ligation reaction may include a reaction temperature of 37 °C and a reaction time of 45 min.

In an exemplary embodiment of the present disclosure, the protected cytosine may be carboxyl cytosine.

In an exemplary embodiment of the present disclosure, the reagents employed for the oxidation reaction may include: TET2 enzyme, TET2 reaction buffer, oxidative supplement, dithiothreitol, oxidative boosting agent, Nuclease-free water, and Fe (II).

In an exemplary embodiment of the present disclosure, the reaction conditions of the oxidation reaction may include: a reaction temperature of 37 °C and a reaction time of 60 min; after the oxidation reaction is completed, a stopping solution is added and incubated at 37 °C for 30 min.

In an exemplary embodiment of the present disclosure, the reagents employed for the deamination reaction may include APOBEC protein, APOBEC reaction buffer, bovine serum protein (BSA), and nuclease-free water.

In an exemplary embodiment of the present disclosure, the reaction conditions of the deamination reaction may include a reaction temperature of 37 °C and a reaction time of 180 min.

In an exemplary embodiment of the present disclosure, the reagents employed for the amplification reaction may also include Index Primer Mix and 2x HiFi HotStart Uracil Mix.

In an exemplary embodiment of the present disclosure, the reaction conditions of the amplification reaction may include: reaction at 98 °C for 30 s; followed by 7 to 16 cycles of reaction at 98 °C for 10 s, reaction at 62 °C for 30 s, and reaction at 65 °C for 60 s; reaction at 65 °C for 5 min. After the amplification reaction is completed, the reactor is maintained at a temperature of 4 °C.

In an exemplary embodiment of the present disclosure, the method for constructing may also include a purification process, which includes any one or more of:
after the extension reaction and before the oxidation reaction, capturing the product obtained by the extension reaction with magnetic beads, and washing the product of the extension reaction captured by the magnetic beads with ethanol and water;
after removing the extended fragment from the double-stranded DNA carrying the protected cytosine and before the deamination reaction, capturing the product obtained after removing the extended fragment with magnetic beads, and washing the product obtained after removing the extended fragment and captured by the magnetic beads with ethanol and water;
after the deamination reaction and before the amplification reaction, capturing the product obtained by the deamination reaction with magnetic beads, and washing the product of the deamination reaction captured by the magnetic beads with ethanol and water.

In an exemplary embodiment of the present disclosure, the method for constructing may further include, after obtaining the DNA methylation library, performing the following procedure,
hybridization reaction;
eluting and amplifying the products of the hybridization reaction;
sequencing the amplified products.

In an exemplary embodiment of the present disclosure, the hybridization reaction includes hybridization reaction employing a hybridization reagent including betaine, dimethyl sulfoxide, and single strand binding protein.

In an exemplary embodiment of the present disclosure, the eluting includes adding a boosting agent including tetramethyl ammonium chloride and formamide.

In an exemplary embodiment of the present disclosure, the tetramethyl ammonium chloride is at a concentration of from 0.5 M to 1.5 M.

In an exemplary embodiment of the present disclosure, the double-stranded DNA fragments are cell-free DNA fragments and/or genomic DNA fragments.

In an exemplary embodiment of the present disclosure, the double-stranded DNA fragment may be obtained according to any method known in the art.

In some embodiments, a double-stranded DNA fragment may be extracted from a sample according to any suitable method known in the art.

In an exemplary embodiment of the present disclosure, the test sample is from whole blood, blood components, plasma, serum, urine, stool, saliva, tissue biopsy, pleural fluid, pericardial fluid, cerebrospinal fluid, or peritoneal fluid.

In an exemplary embodiment of the present disclosure, the method for constructing also includes being used in combination with other omics.

An embodiment of the present disclosure also provides uses of the method for constructing the DNA methylation library in sequencing, detecting the presence or absence of cancer, determining cancer status, monitoring cancer progression, and/or determining cancer classification. An embodiment of the present disclosure also provides a free DNA methylation library, which is obtained by the method for constructing the free DNA methylation library provided above in embodiments of the present disclosure.

An embodiment of the present disclosure also provides a method for sequencing a free DNA methylation, wherein the method for sequencing includes: constructing a free DNA methylation library using the method for constructing a free DNA methylation library provided above in an embodiment of the present disclosure, and then sequencing.

In an exemplary embodiment of the present disclosure, the free DNA sample may be from sample material such as cells, tissues, blood, serum, etc., for example, may be from blood of a patient suspected with tumor.

In an exemplary embodiment of the present disclosure, at least a portion of the free DNA methylation library may be sequenced to obtain sequencing data or sequence reads. In general, any method known in the art may be used to obtain sequence data or sequence reads from a test sample. For example, in one embodiment, sequencing data or sequence reads from cell-free DNA samples may be obtained using next-generation sequencing (NGS). Next-generation sequencing methods include, for example, sequencing by synthesis technology (Illumina), pyrosequencing (454), ion semiconductor technology (Ion Torrent sequencing), single-molecule real time sequencing (Pacific Biosciences), sequencing by ligation (solid-state sequencing) and nanopore sequencing (Oxford Nanopore Technology). In some embodiments, sequencing is massively parallel sequencing using sequencing by synthesis with reversible dye terminators. In other embodiments, sequencing is sequencing by ligation. In other embodiments, sequencing is single-molecule sequencing. In another embodiment, sequencing is paired-end sequencing. Optionally, an amplification step is performed prior to sequencing. In certain embodiments, sequencing includes whole-genome sequencing (or shotgun sequencing) of cfDNA libraries to provide sequence data or sequencing reads representative of the whole genome. In other embodiments, sequencing includes targeted sequencing of cfDNA libraries. For example, sequencing libraries can be enriched for specific target sequences (e.g., a plurality of hybridization probes are used to pull down cfDNA fragments known or suspected to be indicative of cancer) and the target sequences can be sequenced.

In an exemplary embodiment of the present disclosure, after sequencing is completed, the method for the free DNA methylation sequencing may also include analyzing sequencing data or sequencing reads to detect the presence or absence of cancer, determine stage of cancer, monitor progression of cancer, and/or determine cancer classification (e.g., cancer type or cancer tissue of origin). In another embodiment, the sequencing data or sequencing reads may be used to infer the presence or absence of cancer, status of cancer, and/or classification of cancer. For example, sequencing data or sequencing reads may be analyzed to identify methylation profiles indicative of the presence or absence of cancer or to identify one or more mutational signatures indicative of the presence or absence of cancer.

In one embodiment, sequencing data or sequencing reads may be analyzed to detect the presence or absence of cancer, sarcoma, myeloma, leukemia, lymphoma, blastoma, germ cell tumor, or any combination thereof, to determine stages, to monitor progression, and/or to make classification. In some embodiments, the cancer may be an adenocarcinoma. In other embodiments, the cancer may be squamous cell carcinoma. In other embodiments, the cancer is selected from: small cell lung cancer, non-small cell lung cancer, nasopharyngeal carcinoma, colorectal cancer, anal cancer, liver cancer, bladder cancer, cervical cancer, carcinoma of testis, ovarian cancer, gastric cancer, esophageal cancer, head and neck cancer, pancreatic cancer, prostate cancer, kidney cancer, thyroid cancer, melanoma, and breast cancer. In another embodiment, sequencing data or sequencing reads may be analyzed to detect the presence or absence of sarcomas, to determine stages , to monitor progression, and/or to make classification. In certain embodiments, the sarcoma may be selected from: osteosarcoma, chondrosarcoma, leiomyosarcoma, rhabdomyosarcoma, mesotheliosarcoma (mesothelioma), fibrosarcoma, angiosarcoma, liposarcoma, glioma, and astrocytoma. In another embodiment, sequencing data or sequencing reads may be analyzed to detect the presence or absence of leukemia, to determine the stage of leukemia, to monitor the progression of leukemia, and/or to make classification on leukemia. In certain embodiments, the leukemia may be selected from myeloid, granulocytic, lymphoid, lymphocytic and lymphoblastic leukemia. In another embodiment, sequencing data or sequencing reads may be used to detect the presence or absence of lymphoma, determine stage, monitor progression, and/or make classification. In certain embodiments, the lymphoma may be selected from Hodgkin lymphoma and non-Hodgkin lymphoma.

Sequencing may be performed by any method or combination of methods known in the art. For example, known DNA sequencing techniques include, but are not limited to, classical dideoxy sequencing reactions (Sanger method) using labeled terminators or primers and gel separation in plates or capillaries, sequencing by synthesis using reversibly terminated labeled nucleotides, pyrosequencing, 454 sequencing, allele-specific hybridization with labeled oligonucleotide probe libraries, sequencing by synthesis by using allele-specific hybridization with labeled clone libraries, subsequent ligation, real-time monitoring of incorporation of labeled nucleotides in a polymerization step, Polony sequencing, and solid-state sequencing.

A conventional method of performing sequencing is by strand termination and gel separation. Another conventional sequencing method involves chemical degradation of nucleic acid fragments. Hybridization sequencing-based methods are also developed.

Sequencing techniques that can be used in the methods of the present disclosure include, for example, Helicos true single molecule sequencing (tSMS) (Harris T. D. et al. (2008) Science 320: 106-109). Another example of a DNA sequencing technique that can be used in the method of the present disclosure is 454 sequencing (Roche) (Margulies, M et al., 2005, Nature, 437, 376-380). Another example of a DNA sequencing technique that can be used in the methods of the present disclosure is SOLiD technology (Applied Biosystems). Another example of a DNA sequencing technique that can be used in the methods of the present disclosure is ion torrent sequencing.

In some embodiments, the sequencing technique is Illumina sequencing. Illumina sequencing is based on amplification of DNA on solid surfaces using bridged PCR with anchored primers. Genomic DNA can be fragmented, or in the case of cfDNA, fragmentation is not required because the fragment is already short. An adaptor is attached to the 5' and 3' ends of the fragment. DNA fragments attached to the surface of flowing cell channels are extended and bridged for amplification. Fragments become double-stranded, and double-stranded molecules are denatured. A plurality of cycles of solid-phase amplification followed by denaturation can produce clusters of several million single-stranded DNA molecules of the same template of approximately 1000 copies in each channel of the flow cell. Primers, DNA polymerase and four fluorophore-labeled reversible terminated nucleotides are used to perform sequential sequencing. After nucleotide incorporation, a fluorophore is excited with a laser to capture the image and record the identity of the first base. The 3' terminator and fluorophore are removed from each incorporated base, repeating the incorporation, detection and identification steps.

An embodiment of the present disclosure relate to obtaining samples, such as biological samples, such as tissue and/or body fluid samples, from subjects for analysis of a variety of nucleic acids (e.g., a plurality of cfDNA molecules) therein. Samples according to an embodiment of the present disclosure may be collected in any clinically acceptable manner. Any sample suspected to contain a plurality of nucleic acids may be used in conjunction with the methods of the present disclosure. In some embodiments, the sample may include tissue, body fluids, or a combination thereof. In some embodiments, biological samples are collected from healthy subjects. In some embodiments, biological samples are collected from subjects known to have a particular disease or condition (e.g., a particular cancer or tumor). In some embodiments, biological samples are collected from subjects suspected of having a particular disease or disorder.

As used herein, the term "tissue" refers to a large number of attached cellular and/or extracellular matrix materials. Non-limiting examples of tissues commonly used in conjunction with the methods of the present disclosure include skin, hair, nails, endometrial tissue, nasal tissue, central nervous system (CNS) tissue, neural tissue, eye tissue, liver tissue, kidney tissue, placental tissue, breast tissue, gastrointestinal tissue, musculoskeletal tissue, genitourinary tissue, bone marrow, etc., derived from, for example, humans or non-human mammals. Tissue samples according to an embodiment of the present disclosure may be prepared and provided in the form of any tissue sample type known in the art, such as, but not limited to, formalin-fixed paraffin embedded (FFPE), fresh and fresh frozen (FF) tissue samples.

As used herein, the term "bodily fluids" refers to liquid substances originating from subjects (e.g., humans or non-human mammals). Non-limiting examples of body fluids commonly used in conjunction with the methods of the present disclosure include mucus, blood, plasma, serum, serum derivatives, synovial fluid, lymph fluid, bile, sputum, saliva, sweat, tears, sputum, amniotic fluid, menstrual fluid, vaginal fluid, semen, urine, cerebrospinal fluid (CSF), e.g. lumbar or ventricular CSF, gastric juice, liquid samples containing one or more materials derived from nasal, throat, or oral swabs, liquid samples containing one or more materials derived from a lavage process, e.g., peritoneum, stomach, chest, or catheter.

In some embodiments, the sample may include fine needle aspirate or biopsy tissue. In some embodiments, the sample may include a medium containing cells or biological material. In some embodiments, the sample may include a blood clot, for example, a blood clot obtained from whole blood after serum removal. In some embodiments, the sample may include stool. In one preferred embodiment, the sample is taken from whole blood. In one aspect, only a portion of a whole blood sample is used, such as plasma, red blood cells, white blood cells and platelets. In some embodiments, in conjunction with the methods of the present disclosure, the sample is separated into two or more components. For example, in some embodiments, a whole blood sample is separated into plasma, red blood cell, white blood cell and platelet components.

In some embodiments, the sample includes not only a plurality of nucleic acids from the subject from which the sample was taken, but also a plurality of nucleic acids from one or more other organisms, such as viral DNA/RNA present in the subject at the time of sampling.

Nucleic acids may be extracted from the sample according to any suitable method known in the art, and the extracted nucleic acids may be used in conjunction with the methods described herein.

In one exemplary embodiment, cell-free nucleic acids (e.g., cfDNA) are extracted from the sample. cfDNA is a short-base nucleus-derived DNA fragment present in several body fluids (e.g., plasma, stool, urine). Tumor-derived circulating tumor DNA (ctDNA) constitutes a minority of cfDNA, with variations of up to ~ 50% in certain cases. In some embodiments, ctDNA varies according to tumor stage and tumor type. In some embodiments, ctDNA varies from about 0.001% to about 30%, e.g. from about 0.01% to about 20%, e.g. from about 0.01% to about 10%. Covariates of ctDNA are not fully understood, but appear to be positively associated with tumor type, tumor size, and tumor stage. As Bettegowda et al., Sci Trans Med, 2014; Newmann et al., Nat Med, 2014. Despite the challenges of low numbers of ctDNA in cfDNA, tumor variants have been identified in ctDNA in a wide range of cancers. Such as Bettegowda et al., Sci Trans Med, 2014. In addition, cfDNA analysis is less invasive than tumor biopsy, and analytical methods, such as sequencing, enable an identification of subclonal heterogeneity. Analysis of cfDNA also showed to provide more consistent whole-genome sequencing coverage compared to tumor tissue biopsies. In some embodiments, a plurality of cfDNAs are extracted from a sample in a manner that reduces or eliminates blending of cfDNA and genomic DNA. For example, in some embodiments, the sample is processed to isolate a plurality of cfDNAs therein in less than about 2 hours, such as less than about 1.5, 1, or 0.5 hours.

A non-limiting example of a procedure for preparing nucleic acids from a blood sample is as follows. Blood may be collected in 10 mL EDTA tubes (e.g., BD VACUTAINER Series Products from Becton Dickinson, Franklin Lake, N.J.) or in collection tubes suitable for cfDNA isolation (e.g., Cell-Free DNA BCT Series Products from Streck, Omaha, Nebraska). May be used to minimize contamination by chemical fixation of nucleated cells, but little contamination from genomic DNA is observed when the sample is processed in 2 hours or less, as is the case in some embodiments of the present method. Starting with a blood sample, plasma can be extracted by centrifugation, e.g. at 3000 rpm for 10 min at room temperature with deceleration braking. Plasma could then be transferred in 1 ml aliquots into 1.5 ml tubes and centrifuged again at 7000 rpm for 10 min at room temperature. A supernatant is then transferred to a new 1.5 ml tube. At this stage, samples can be stored at 80 °C. In certain embodiments, samples may be stored at the plasma stage for later processing, as plasma may be more stable than extracted cfDNA when being storinged.

Plasma DNA can be extracted using any suitable technique. For example, in some embodiments, plasma DNA may be extracted using one or more commercially available assays, such as the QIAmp Circulating Nucleic Acids Kit Series (Qiagen n n v., Venlo Netherlands). In certain embodiments, the following improved elution strategies may be used. DNA can be extracted using, for example, the QIAmp Circulating Nucleic Acids Kit, following the manufacturer's instructions (maximum allowable plasma volume of 5 mL per column). If cfDNA is extracted from plasma, where blood is collected in Streck tubes, a reaction time with protease K can be doubled, from 30 to 60 minutes. Preferably, the largest possible volume (i.e., 5 mL) should be used. In various embodiments, two-step elution can be used to maximize cfDNA yield. First, DNA could be eluted using 30 µL AVE buffer per column. To increase a cfDNA concentration, a minimum amount of buffer required to fully cover a membrane can be used in elution. By reducing a dilution of a small amount of buffer, downstream drying of the sample can be avoided to prevent meltment or material loss of double-stranded DNA. Subsequently, above 30 µL of buffer is eluted per column. In some embodiments, a second elution may be used to increase DNA yield.

An embodiment of the present disclosure also relates to computer systems and devices.

Aspects of the present disclosure described herein may be implemented using any type of computing device, such as a computer, including a processor, such as a central processing unit, or any combination of computing devices, each of which implements at least part of the process or method. In some embodiments, the systems and methods described herein may be performed with handheld devices such as smart tablet computer, smart phones or specialized devices manufactured for the system.

The methods of the present disclosure may be performed using software, hardware, firmware, hardwires, or any combination thereof. Features that implement functions may also be physically located at various locations, including distributing at different physical locations to implement parts of functions to (e.g., imaging devices in one room, host workstations in another room, or in separate buildings, e.g., via wireless or wired connections).

Processors adapted to execute computer programs include, for example, general and special microprocessors, and any one or more processors of any type of digital computer. Typically, the processor will receive instructions and data from either read-only memory or random access memory, or both. Basic elements of a computer are a processor for executing instructions and one or more storage devices for storing instructions and data. Typically, computer will also include or be operably coupled to one or more mass storage devices for storing data, such as a disk, magneto-optical disc, or optical disc, to receive data from or transfer data to the mass storage device, or both. An information carrier suitable to contain computer program instructions and data includes all forms of non-volatile memory, including, for example, semiconductor memory devices (e.g., EPROM, EEPROM, solid state drive (SSD) and flash memory devices); disks (e.g. internal hard disks or removable disks); magneto-optical disk; and compact discs (such as CDs and DVD discs). Processor and memory may be supplemented by or incorporated into dedicated logic circuits.

To provide interaction with the user, the subject matter herein may be implemented on a computer having I/O devices for displaying information to the user, such as CRT, LCD, LED or projection devices, and input or output devices, such as keyboards and fixed-point devices (such as a mouse or trackball), through which the user may provide input to the computer. Other types of devices may also be used to provide interaction with the user. For example, a feedback provided to the user may be any form of sensory feedback (e.g., visual feedback, auditory feedback, or tactile feedback), and input from the user may be received in any form, including auditory, phonetic, or tactile input.

The subject matter described herein may be implemented in a computing system including a back-end component (e.g., a data server), a middleware component (e.g., an application server), or a front-end component (e.g., a client computer with a graphical user interface or web browser through which an user may interact with the implementation of the subject matter described herein), or any combination of the back-end, middleware, and front-end components. The components of the system can be interconnected over a network, such as a communication network, through digital data communication of any form or medium. For example, a reference dataset may be stored at a remote location, and computers may communicate over a network to access the reference dataset for comparison purposes. However, in other embodiments, the reference dataset may be stored locally within the computer, and the computer accesses the reference dataset within the CPU for comparison purposes. Examples of communication networks include, but are not limited to, cellular networks (e.g., 3G or 4G), local area networks (LANs), and wide area networks (WANs), such as the Internet.

The subject matter described herein may be embodied as one or more computer program products, such as one or more computer programs tangibly embodied in an information carrier (e.g., non-transitory computer readable media) for performing or controlling operations thereof by a data processing device (e.g., a programmable processor, computer or a plurality of computers). A computer program (also known as a program, software, software application, app, macro, or code) can be written in any form of programming language, including a compiled or interpreted language (e.g., C, C ++, Perl), and it can be deployed in any form, including as a stand-alone program or as a module, component, subroutine, or other unit suitable for use in a computing environment. The systems and methods of the present disclosure may include instructions written in any suitable programming language known in the art, including but not limited to C, C ++, Perl, Java, ActiveX, HTML5, Visual Basic, or JavaScript.

A computer program does not necessarily correspond to a file. Programs can be stored in files or a portion of files that hold other programs or data, in a single file dedicated to the program in question, or in a plurality of collaborative files (e.g., files that store one or more modules, subprograms, or code sections). Computer programs may be deployed to execute on one computer or a plurality of computers at one location, or distributed across a plurality of locations and interconnected over a communication network.

A file may be a digital file, for example, stored on a hard disk drive, SSD, CD, or other tangible non-transient media. A file can be sent from one device to another over the network (e.g., as packets sent from a server to a client, e.g., through a network interface card, modem, wireless card, etc.).

Writing files according to the present disclosure relate to converting tangible non-transient computer-readable media, for example, by adding, removing, or rearranging particles (for example, converting a net charge or dipole moment into a magnetization mode by a read/write head), which then represents a new collocation of information about an objective physical phenomenon that is required by and useful to the user. In some embodiments, writing relates physical conversion of material in a tangible, non-transient computer-readable medium (e.g., having certain optical properties such that the optical read/write device can then read new and useful information configurations, e.g., burn CD-ROMs). In some embodiments, writing a file includes transforming a physical flash device, such as a NAND flash device, and storing information by transforming physical elements in a memory cell array made of floating gate transistors. A method of writing a file is well known in the art and may be invoked manually or automatically for example by a program or by a save command from software or a write command from a programming language.

Suitable computing devices typically include mass memory, at least one graphical user interface, at least one display device, and typically include communication between devices. Mass memory shows a computer readable medium, i.e. a computer storage medium. Computer storage media may include volatile, non-volatile, removable, and non-removable media implemented in any method or technique for storing information, such as computer-readable instructions, data structures, program modules, or other data. Examples of computer storage media include RAM, ROM, EEPROM, flash memory or other storage technology, CD-ROM, digital multifunction disk (DVD) or other optical storage, cassette tape, magnetic tape, disk storage or other magnetic storage device, radio frequency identification (RFID) tag or chip, or any other media that can be used to store required information and can be accessed by a computing device.

The functions described herein may be implemented using software, hardware, firmware, hardwiring, or any combination thereof. Any software can be physically located at different locations, including distributed at different physical locations to implement parts of functions.

As recognized by those skilled in the art, a computer system for implementing some or all of the inventive methods may include one or more processors (e.g., a central processing unit (CPU), a graphics processing unit (GPU), or both), a main memory, and a static memory, which communicate with each other over a bus, if necessary or most suitable for the execution of the method of the present disclosure.

Processors typically include chips, such as single-core or multi-core chips, to provide a central processing unit (CPU). Intel or AMD chips can provide a process.

Memory may include one or more machine-readable devices on which one or more sets of instructions (e.g., software) are stored that, when executed by a processor of any of the computers, which may implement some or all of the methods or functions described herein. Softwares may also reside fully or at least partially in the main memory and/or processor during execution by the computer system. Preferably, each computer includes non-transient memory, such as a solid-state drive, flash drive, disk drive, hard disk drive, and the like.

While in an exemplary embodiment, the machine-readable device may be a single medium, the term "machine-readable device" should include a single medium or a plurality of media (e.g., centralized or distributed databases, and/or associated caches and servers) that store one or more sets of instructions and/or data. These terms are also to be understood to include any medium or media capable of storing, encoding, or preserving a set of instructions executed by a machine and causing the machine to execute any one or more of the methods of the present disclosure. Accordingly, these terms are to be understood to include, but are not limited to, one or more solid-state memories (e.g., user identity module (SIM) cards, secure digital cards (SD cards), micro-SD cards or solid-state drives (SSD)), optical and magnetic media, and/or any other tangible storage media.

Computers of the present disclosure typically include one or more I/O devices, such as one or more video display devices (e.g., liquid crystal display (LCD) or cathode ray tube (CRT)), alphanumeric input devices (e.g., keyboards), cursor control devices (e.g., mice), disk drive devices, signal generation devices (e.g., speakers), touch screens, accelerometers, microphones, cellular radio frequency antennas, and network interface devices, which may be, e.g.,

Any software can be physically located at different locations, including distributed at different physical locations to implement parts of functions.

Further, the system of the present disclosure may include reference data. Any suitable genomic data can be stored for use in the system. Examples include, but are not limited to: comprehensive, multidimensional maps of key genomic changes in major types and subtypes of cancers from the Cancer Genome Atlas (TCGA); catalogue of genomic abnormalities of the International Cancer Genome Consortium (ICGC); catalogue of somatic mutations in cancer from COSMIC; recent constructions of the human genome and other popular model organisms; latest reference SNPs from dbSNPs from the 1000 Genomes Project and gold-labeled indels of the Broad Institute annotated exome capture kits from Illumina, Agilent, Nimblegen and Ion Torrent; transcript annotation; small amount of test data for piping testing (for example, for new users).

In some embodiments, the data is available in the context of a database included in the system. Any suitable database structure can be used, including relational databases, object-oriented databases, and so on. In some embodiments, the reference data is stored in a relational database, such as a "non-unique SQL" (NoSQL) database. In some embodiments, a graph database is included in the system of the present disclosure. It should also be understood that the term "database" used here is not limited to a single database; instead, a system can contain a plurality of databases. For example, according to an embodiment of the present disclosure, the database may include two, three, four, five, six, seven, eight, nine, ten, fifteen, twenty, or more individual databases, including any integer number of databases thereof. For example, one database may contain public reference data, a second database may contain test data from patients, a third database may contain data from healthy subjects, and a fourth database may contain data from sick subjects with known conditions or disorders. It should be understood that the method described herein also takes into account any other configuration of the database with respect to the data contained therein.

Whole genome sequencing has a large amount of data and high cost, and targeted sequencing can be used to solve this problem. Targeted sequencing requires enrichment and sequencing of some regions of interest in the genome employing a plurality of amplification or hybridization capture. During the preparation of methylated libraries, unmethylated C bases will be converted into U bases, and after amplification and enrichment, U bases becomes A bases, resulting in an increase in the proportion of A and T bases, which affects a probe capture efficiency.

An embodiment of the present disclosure also provides a method for DNA hybridization and capture, the method for DNA hybridization and capture including:
hybridization reaction;
eluting and amplifying the products of the hybridization reaction;
sequencing the amplified products.

In an exemplary embodiment of the present disclosure, the hybridization reaction includes hybridization reaction employing a hybridization reagent including betaine, dimethyl sulfoxide, and a single strand binding protein.

An exemplary embodiment of the present disclosure can improve an efficiency of a hybridization reaction by introducing a complex of betaine, dimethyl sulfoxide and single strand binding protein into a hybridization reagent.

Herein, in the hybridization reaction, the methylated library needs to undergo high-temperature denaturation, and then a single-stranded DNA molecule hybridizes with a probe, for example, at a hybridization temperature of 60 °C. If the single-stranded DNA molecule is renatured, the hybridization efficiency of the probe will be affected. An embodiment of the present disclosure employs single stranded binding protein SSB to bind a single-stranded DNA molecule, and a tetramer formed by the SSB specifically binds 8-16 bases, avoiding a renaturation of the single-stranded DNA, and effectively improving the hybridization efficiency.

In an exemplary embodiment of the present disclosure, the hybridization reagent may also include Cot-1 DNA. Cot-1 DNA is a kind of placental DNA, with a size mainly in a range of 50 to 300 bp, and rich in repeated DNA sequences, which can effectively block the repeated DNA sequences in the target region and reduce non-specific hybridization.

In an exemplary embodiment of the present disclosure, the eluting includes adding a boosting agent including tetramethyl ammonium chloride and formamide.

An exemplary embodiment of the present disclosure can significantly improve the specificity of unbalanced library hybridization by adding tetramethyl ammonium chloride and formamide as boosting agents to the eluent.

Herein, since the ratio of A and T bases of the DNA strands in the methylated library increases after methylation transformation, tetramethyl ammonium chloride in the eluent can increase a melting temperature of DNA strands rich in A and T bases, and increase the TM value of a region rich in A and T bases in the DNA strand, making it close to the TM value of the DNA strand with a normal ratio of A and T bases. Test results of gradient concentrations of tetramethyl ammonium chloride (0.1 M, 0.5 M, 1M, 1.5 M, 2 M) showed that the concentration of tetramethyl ammonium chloride was better at 1M. In the elution step, the eluent was incubated to 48 °C, which could effectively wash away the non-specific hybridization products, and ensure that the specific hybridization products rich in A and T bases remained.

Formamide can reduce the TM value of the DNA strand, and every increase of 1% formamide can reduce the TM value of about 0.7 °C. After incubation to 48 °C, the elute containing 5% formamide can enhance the specificity of hybridization to a greater extent, and reduce the loss of hybridization products in the target region to a lower extent.

In an exemplary embodiment of the present disclosure, the tetramethyl ammonium chloride may be at a concentration of from 0.5 M to 1.5 M.

An embodiment of the present disclosure also provides a kit for constructing a DNA methylation library, the kit is used for performing the method for constructing the DNA methylation library provided above in embodiments of the present disclosure.

The kit includes: the double-stranded adapter sequence, ligation reagent for the adapter sequence, an extension reagent, an oxidative protection reagent, a reagent for removing extended fragment, a deamination reagent, and an amplification reagent.

In an exemplary embodiment of the present disclosure, the double-stranded adapter sequence includes a first double-stranded adapter sequence which is a splint sequence formed by a first oligonucleotide sequence and a second oligonucleotide sequence, and a second double-stranded adapter sequence which is a splint sequence formed by a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than that the third oligonucleotide sequence;
overhangs of the second oligonucleotide sequence and the fourth oligonucleotide sequence can be complementary to and paired with the single-stranded DNA.

In an exemplary embodiment of the present disclosure, the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4.

In an exemplary embodiment of the present disclosure, the ligation reagent for the adapter sequence may include an aqueous solution of polyethylene glycol, DNA ligase buffer, dithiothreitol, adenine nucleoside triphosphate, polynucleotide kinase, and DNA ligase.

In an exemplary embodiment of the present disclosure, the extension reagents may include Klenow reaction buffer, a mixture of dNTP (not including dTTP) and dUTP, an aqueous solution of Tween 20, and Klenow Fragment.

In an exemplary embodiment of the present disclosure, a molar ratio of dNTP to dUTP is 0.5:1 to 2:1.

In an exemplary embodiment of the present disclosure, the oxidative protection reagent may include: TET2 enzyme, TET2 reaction buffer, oxidative supplement, dithiothreitol, oxidative boosting agent, nuclease-free water, and Fe (II).

In an exemplary embodiment of the present disclosure, the reagent for removing extended fragment may include an UDG enzyme.

In an exemplary embodiment of the present disclosure, the deamination reagent may include APOBEC protein, APOBEC reaction buffer, bovine serum protein, and nuclease-free water.

In an exemplary embodiment of the present disclosure, the amplification reagent may also include Index Primer Mix and HiFi HotStart Uracil Mix.

In an exemplary embodiment of the present disclosure, the kit may also include an operation manual that may include operating the kit in accordance with the method for constructing the free DNA methylation library provided above in an embodiment of the present disclosure.

An embodiment of the present disclosure also provides a kit for hybridization and capture, which is used for performing the method for DNA hybridization and capture described in the embodiment of the present disclosure.

In an exemplary embodiment of the present disclosure, the kit for hybridization and capture includes: a hybridization reagent employed for the hybridization reaction and a boosting agent employed for the eluting.

In an exemplary embodiment of the present disclosure, the hybridization reagent includes betaine, dimethyl sulfoxide, and single strand binding protein.

In an exemplary embodiment of the present disclosure, the eluting includes adding a boosting agent including tetramethyl ammonium chloride and formamide.

In an exemplary embodiment of the present disclosure, the tetramethyl ammonium chloride is at a concentration of from 0.5 M to 1.5 M.

### Example 1

The present example provides a method for constructing a free DNA methylation library and a method for sequencing a free DNA methylation, including:
(1) preparation of standard samples: customizing cfDNA standard samples from GeneWell, including fully methylated standard samples (that is, all of C bases in the sequence of the standard samples were methylated) and fully unmethylated standard samples (that is, none of C bases in the sequence of the standard products is methylated), mixing the standard samples according to a certain ratio into 1 µg standard samples with a 50% methylation rate, and then adding the standard samples with a 50% methylation rate to a 0.6 mL PCR tube for storing;
(2) denaturation: taking 1 ng, 10 ng, 50 ng of the standard samples with 50% methylation rate to 0.2 mL tubes respectively and labeling as Samples 1, 2, 3, placing Samples 1, 2, 3 in a PCR instrument and incubating at 95 °C for 5 min, then immediately placing the PCR tubes on ice for cooling, and standing for 2 min, so that the DNA in the standard samples being fully melted into single-stranded DNA;
(3) ligation reaction: thawing the reagents shown in the following table, mixing upside down, and instantaneously centrifuging, and then adding the reagents sequentially to the PCR tube containing 16 µL of the product obtained in step (2), wherein this step was operated on ice; gently blowing or shaking the PCR tube to mix the reaction solution in the PCR tube, and instantaneously centrifuging the reaction solution to the bottom of the tube, after the centrifugation completed, putting the PCR tube into the PCR instrument, and performing the ligation reaction at 37 °C, for 45 min.

**Table 1**

| Reagent name | Volume (µL) |
|---|---|
| First adapter sequence (20 µM) | 1 |
| Second adapter sequence (20 µM) | 1 |
| PEG 8000 (50%) | 15 |
| 10× T4 DNA ligase buffer | 5 |
| DTT (1 M) | 1 |
| ATP (10 mM) | 5 |
| T4 PNK (10 U/µL) | 1 |
| T4 DNA ligase | 5 |
| Total | 50 |

Herein, the adapter sequence was formed by annealing 4 oligonucleotides, and sequences of the oligonucleotides were shown in Table 2:

**Table 2**

| Number of oligonucleotide | Sequence (5 '-3') |
|---|---|
| 1 | ACACTCTTTCCCTACACGACGCTCTTCCGATCT (as set forth in SEQ ID NO.1 in the sequence listing) |
| 2 | NNNNNNNAGATCGGAAGAGCGTCGTGTAGGGAAAGAGTGT (as set forth in SEQ ID NO.2 in the sequence listing) |
| 3 | AGATCGGAAGAGCACACGTCTGAACTCCAGTC (as set forth in SEQ ID NO.3 in the sequence listing) |
| 4 | GACTGGAGTTCAGACGTGTGCTCTTCCGATCTNNNNNNN (as set forth in SEQ ID NO.4 in the sequence listing) |

Annealing steps for adapter sequences:
step 1: resuspending oligonucleotides 1 to 4 with amino modifications, respectively, to obtain a solution with a concentration of 100 µM and a volume of 100 µL, respectively;
step 2: preparing buffer reagent 100 µL, reagent composition:
   10 mM (Tris (hydroxymethyl) aminomethane)-HCl) buffer at pH 7.5, 2 mM EDTA, 50 mM NaCl;
step 3: placing 10 µL oligonucleotide 1 and oligonucleotide 2 respectively in a first PCR tube, placing 10 µL oligonucleotide 3 and oligonucleotide 4 in a second PCR tube, and adding 80 µL buffer reagent to the above PCR tubes, respectively, fully mixing, and centrifuging for 10 s;
step 4: placing the above PCR tubes in a PCR instrument and denaturing at 95 °C for 10 min;
step 5: after the reaction completed, turning off the PCR instrument directly, and taking out the PCR tubes when the temperature drops to room temperature.

A product in the first PCR tube was a first adapter sequence as shown in FIG. 2A, and a product in the second PCR tube was a second adapter sequence as shown in FIG. 2B.
(4) extension reaction: after the previous step of reaction being completed, immediately preparing the following reaction system in a PCR tube according to the table below, gently blowing or shaking the PCR tube to mix the reaction solution in the PCR tube, and instantaneously centrifuging the reaction solution to the bottom of the tube, after the centrifugation completed, putting the PCR tubes into the PCR instrument for extension reaction; wherein, a heat cap was at a temperature of 100 °C, the extension reaction was at a reaction temperature of 20 °C, for a reaction time of 15 min, and the PCR tube was maintained at a temperature of 4 °C after the reaction is completed;

**Table 3**

| Reagent name | Volume (µL) |
|---|---|
| Product of Step (3) | 50 |
| 10× Klenow reaction buffer | 6.5 |
| A mixture of dNTP (not including dTTP) and dUTP (stock solution at a concentration of 10mM) | 2 (dNTP:dUTP at a molar ratio of 1:1) |
| Tween 20 (1%) | 4.5 |
| Klenow Fragment | 2 |
| Total | 65 |

(5) purification of product: adding 80 µL of Novozyme magnetic beads for purification to the product of step (4), fully mixing, standing at room temperature for 5 min, placing on a magnetic rack for about 5 min to allow the magnetic beads being completely adsorbed and the solution being clarified, and carefully removing the supernatant; adding 200 µL of freshly prepared 80 volume % ethanol for rinsing, incubating at room temperature for 30 s to 60 s, removing the supernatant carefully, repeating once; after the magnetic beads was dry, adding 22 µL of ultrapure water to elute, leaving at room temperature for 3 min and placing on a magnetic rack, after the solution was clarified, pipetting 20 µL of supernatant for use;
(6) oxidation reaction: thawing the reagents shown in the following table, mixing upside down, and performing instantaneous centrifugation, and then adding the reagents sequentially to a PCR tube containing 20 µL of the product obtained in step (5), wherein this step was operated on ice; gently blowing or shaking the PCR tube to mix the reaction solution in the PCR tube, and instantaneously centrifuging, then adding 5 µL Fe (II) solution, fully mixing again and briefly centrifuging, after the centrifugation was completed, putting the PCR tube into the PCR instrument for oxidation reaction, wherein, the oxidation reaction was at a reaction temperature of 37 °C, for a reaction time of 60 min; after the reaction was completed, leaving on ice, adding 1 µL of stop solution and incubating at 37 °C for 30 min;

**Table 4**

| Reagent name | Volume (µL) |
|---|---|
| Product of Step (5) | 20 |
| TET2 reaction buffer | 10 |
| Oxidative supplements | 1 |
| DTT | 1 |
| Oxidative boosting agent | 1 |
| TET2 enzyme | 4 |
| Nuclease-free water | 8 |
| Total | 45 |

In this example, the step employed the Enzymatic Methyl-seq Conversion Module Kit (Cat. No. E7125) of NEB Company;
(7) UDG digestion reaction: after the previous step of reaction was completed, immediately preparing the following reaction system in the PCR tubes according to the table below, gently blowing or shaking the PCR tubes to mix the reaction solution in the PCR tubes, and instantaneously centrifuging the reaction solution to the bottom of the tube, after the centrifugation was completed, putting the PCR tube into the PCR instrument for digestion reaction; wherein, a first digestion stage was at a reaction temperature of 37 °C, for a reaction time of 20 min; a second digestion stage was at a reaction temperature of 50 °C for a reaction time of 5 min;

**Table 5**

| Reagent name | Volume (µL) |
|---|---|
| Product of Step (6) | 46 |
| UDG enzyme (1U/µL) | 1 |
| Total | 46 |

(8) purification of product: adding 80 µL of Novozyme magnetic beads for purification to the product of step (7), fully mixing, standing at room temperature for 5 min, placing on a magnetic rack for about 5 min to allow the magnetic beads being completely adsorbed and the solution being clarified, and carefully removing the supernatant; adding 200 µL of freshly prepared 80 volume% ethanol for rinsing, incubating at room temperature for 30 s to 60 s, removing the supernatant carefully, repeating once; after the magnetic beads is dry, adding 18 µL of ultrapure water to elute, leaving at room temperature for 3 min and placing on a magnetic rack, after the solution was clarified, absorbing 16 µL of supernatant for use;
(9) deamination reaction: adding 4 µL formamide to the product of step (8), fully mixing, centrifuging briefly, incubating at 85 °C for 10 min, immediately placing on ice after the reaction was completed, and preparing the reaction system shown in the following table in a PCR tube; gently blowing the PCR tube to mix the reaction solution in the PCR tube, and centrifuging briefly; after the centrifugation was completed, putting the PCR tube into the PCR instrument for a deamination reaction, wherein a heat cap was at a temperature of 75 °C, the deamination reaction was at a temperature of 37 °C, and for a reaction time of 180 min;

**Table 6**

| Reagent name | Volume (µL) |
|---|---|
| Product of Step (8) | 16 |
| Formamide | 4 |
| Nuclease-free water | 68 |
| APOBEC reaction buffer | 10 |
| BSA | 1 |
| APOBEC protein | 1 |
| Total | 100 |

In this example, the step employed the Enzymatic Methyl-seq Conversion Module Kit (Cat. No. E7125) of NEB Company;
(10) purification of product: adding 100 µL of Novozyme magnetic beads for purification to the product of step (9), fully mixing, standing at room temperature for 5 min, placing on a magnetic rack for about 5 min to allow the magnetic beads being completely adsorbed and the solution being clarified, and carefully removing the supernatant; adding 200 µL of freshly prepared 80 volume % ethanol for rinsing, incubating at room temperature for 30 s to 60 s, removing the supernatant carefully, repeating once; after the magnetic beads is dry, adding 22 µL of ultrapure water to elute, leaving at room temperature for 3 min and placing on a magnetic rack, after the solution is clarified, pipetting 20 µL of supernatant for use;
(11) amplification of product: thawing Index Primer Mix and KAPA HiFi HotStart Uracil and mixing upside down, and preparing the reaction system as shown in Table 7 in a sterilized PCR tube; gently blowing the PCR tube to mix the reaction solution in the PCR tube, and centrifuging briefly, after the centrifugation was completed, putting the PCR tube into the PCR instrument for amplification reaction, a reaction conditions of the amplification reaction was shown in Table 8;

**Table 7**

| Reagent name | Volume (µL) |
|---|---|
| Product of Step (10) | 20 |
| Index Primer Mix | 5 |
| 2× HiFi HotStart Uracil Mix | 25 |
| Total | 50 |

**Table 8**

| Temperature | Time | Number of cycles |
|---|---|---|
| 98 °C | 30 s | 1 |
| 98 °C | 10s | Determined by the amount of DNA input |
| 62 °C | 30 s | |
| 65 °C | 60 s | |
| 65 °C | 5min | 1 |
| 4 °C | Holding | |

The primer for PCR amplification includes 2 primers, and the primers may include any one of the following labeled sequences numbered 1-16, which can be used to distinguish samples. The labeled sequences were as follows:

**Table 9**

| Number of sample label | i5 sample label (Novaseq v1.0, Hiseq2000/2500, Miseq) | i5 sample label (Novaseq v1.5, Hiseq3000/4000, Miniseq) | i7 sample label (all ill µMina systems) |
|---|---|---|---|
| 1 | CGACCATT | AATGGTCG | GCCTATCA |
| 2 | GATAGCGA | TCGCTATC | CTTGGATG |
| 3 | AATGGACG | CGTCCATT | AGTCTCAC |
| 4 | CGCTAGTA | TACTAGCG | CTCATCAG |
| 5 | TCTCTAGG | CCTAGAGA | TGTACCGT |
| 6 | ACATTGCG | CGCAATGT | AAGTCGAG |
| 7 | TGAGGTGT | ACACCTCA | CACGTTGT |
| 8 | AATGCCTC | GAGGCATT | TCACAGCA |
| 9 | CTGGAGTA | TACTCCAG | CTACTTGG |
| 10 | GTATGCTG | CAGCATAC | CCTCAGTT |
| 11 | TGGAGAGT | ACTCTCCA | TCCTACCT |
| 12 | CGATAGAG | CTCTATCG | ATGGCGAA |
| 13 | CTCATTGC | GCAATGAG | CTTACCTG |
| 14 | ACCAGCTT | AAGCTGGT | CTCGATAC |
| 15 | GAATCGTG | CACGATTC | TCCGTGAA |
| 16 | AGGCTTCT | AGAAGCCT | TAGAGCTC |

(12) purification of product: adding 45 µL of Novozyme magnetic beads for purification to the product of step (11), fully mixing, standing at room temperature for 5 min, placing on a magnetic rack for about 5 min to allow the magnetic beads being completely adsorbed and the solution being clarified, and carefully removing the supernatant; adding 200 µL of freshly prepared 80 volume % ethanol for rinsing, incubating at room temperature for 30 s to 60 s, removing the supernatant carefully, repeating once; after the magnetic beads was dry, adding 32 µL of ultrapure water to elute, leaving at room temperature for 3 min and then placing on a magnetic rack, after the solution was clarified, pipetting 30 µL of supernatant for use;
(13) on-machine sequencing: diluting the methylation library obtained in step (12) to 1 ng/µL, taking out 1 µL and employing Agilent 4200 Tapestation system (Agilent, USA) for detection; in addition, taking out 1 µL for qPCR detection, and determining a concentration on the machine according to the detection results. According to the concentration obtained in the previous step, the library was diluted to a concentration meeting the requirement for loading onto the instrument (2 nmol), and PE150 sequencing was carried out on Novaseq sequencing platform of Illumina Company, with the data volume of each sample being 1.5 G.

### Example 2

The difference between this example and Example 1 was that a quality control was not added to the standard samples of Example 1, while a quality control was added to the standard samples of Example 2: taking 1 ng, 10 ng, 50 ng of the standard samples with 50% methylation rate to a 0.2 mL PCR tube, respectively, and labeling as Samples 4, 5, and 6, and adding 1 µL of 0.1 ng/µL of pUC19 plasmid DNA and 1 µL of 2 ng/µL of Lambda DNA to Samples 4, 5, and 6, respectively, as quality control, and employing Lamda DNA and pUC19 plasmid DNA as Control DNA, the amount of which was different according to the data amount of the sample cfDNA.

The pUC19 plasmid DNA employed CpG methylated pUC19, and all cytosine (C base) of the pUC19 plasmid DNA was completely methylated. Therefore, theoretically, the methylation rate of the pUC19 plasmid DNA was 100%, which was used to indicate whether the protection of methylated cytosine was effective. When the methylation rate of the pUC19 plasmid DNA was greater than 99%, it could be regarded as successful protection of methylation. At the same time, the added 1 µL 2 ng/µL Lamda DNA employed Unmethylated lambda DNA, which was completely unmethylated, that is, all contained cytosines (C bases) were completely unmethylated. The methylation rate of Lambda DNA could indicate whether the conversion of unmethylated C bases was effective in experiments. If the methylation rate of the final analyzed DNA was less than 1%, the test could be considered a success. Therefore, Lambda DNA and pUC19 plasmid DNA added in Samples 4, 5 and 6 could be used as internal reference DNA.

### Example 3

The difference between this example and Example 1 was only that the modification groups at the ends of the first adapter sequence and the second adapter sequence were different, as follows:
1) sample 7: the first adapter sequence employed the adapter as shown in FIG. 3A, and the second adapter sequence employed the adapter as shown in FIG. 3B;
2) sample 8: the first adapter sequence employed the adapter as shown in FIG. 4A, and the second adapter sequence employed the adapter as shown in FIG. 4B;
3) sample 9: the first adapter sequence employed the adapter as shown in FIG. 5A, and the second adapter sequence employed the adapter as shown in FIG. 5B.

### Example 4

The difference between this example and Example 1 was only that the molar ratio of dNTP (not including dTTP) to dUTP was different in the extension reaction of Step (4), as follows:
1) sample 10: dNTP: dUTP at a molar ratio of 0.25: 1;
2) sample 11: dNTP: dUTP at a molar ratio of 0.5: 1;
3) sample 12: dNTP: dUTP at a molar ratio of 2: 1;
4) sample 13: dNTP: dUTP at a molar ratio of 4: 1.

### Comparative Example 1

In this Comparative Example, the method for constructing sulfite single-strand library was employed to construct a free methylation library: Accel-NGS ^{®} Methyl-Seq DNA Library Kit purchased from Swift Bioscience was employed, and the specific operation was detailed in the kit operation manual. 1ng, 10ng, 50ng of standard samples with 50% methylation rate were taken to 0.2 mL PCR tubes as test samples, and labeled as Samples 14, 15, 16.

### Comparative Example 2

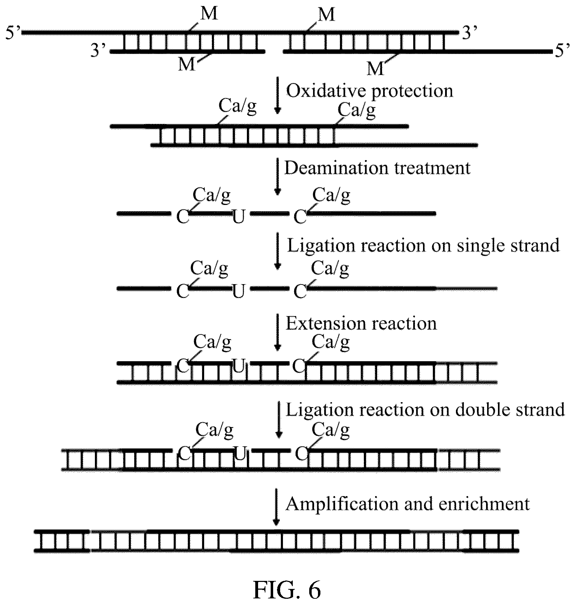

In this Comparative Example, the enzymatic conversion double-strand library was employed to construct a free DNA methylation library. FIG. 6 is a schematic flowchart of a method for constructing enzyme conversion double-stranded library of this Comparative Example.

1 ng, 10 ng, 50 ng of standard samples with 50% methylation rate was taken to 0.2 mL PCR tubes, respectively, labeled as Samples 17, 18, 19, and according to the flow as shown in Fig.6, TET2 enzyme and the like was used to directly oxidize and protect cfDNA with methylation modified cytosine, followed by deamination treatment, the unmethylated C base was deaminated to form an U base, and the double-stranded DNA was melted into a single-stranded DNA, and then a ligation reaction was allowed to occur between one end of the single-stranded DNA and an adapter sequence, and the double-stranded DNA was formed through a primer extension reaction, and then a ligation reaction was allowed to occur between both ends of the double-stranded DNA and the adapter sequence respectively, and the subsequent amplification and enrichment formed a free DNA methylation library.

The sequencing results of samples in the above Examples and the Comparative Examples were shown in Tables 10 to 12.

**Table 10**

| Number of sample | pUC19 methylation rate | LambdaDNA methylation rate | note |
|---|---|---|---|
| 4 | 99.10% | 0.20% | Example 2 |
| 5 | 99.50% | 0.05% | Example 2 |
| 6 | 99.70% | 0.06% | Example 2 |

It could be seen that the methylation rate of pUC19 plasmid DNA in samples 4 to 6 was greater than 99%, and the methylation rate of Lambda DNA was significantly lower than 1%, indicating that the methylation protection of samples 4 to 6 and the conversion of unmethylated C bases were all successful. Therefore, the method for constructing a free DNA methylation library provided by the embodiment of the present disclosure was feasible.

**Table 11**

| Number of sample | Initial input amount of sample (ng) | Yield of library (ng) | note |
|---|---|---|---|
| 1 | 1 | 800 | Example 1 |
| 2 | 10 | 1100 | Example 1 |
| 3 | 50 | 1200 | Example 1 |
| 4 | 1 | 820 | Example 2 |
| 5 | 10 | 1090 | Example 2 |
| 6 | 50 | 1150 | Example 2 |
| 7 | 10 | 1080 | Example 3 |
| 8 | 10 | 1130 | Example 3 |
| 9 | 10 | 1096 | Example 3 |
| 10 | 10 | 1065 | Example 4 |
| 11 | 10 | 1200 | Example 4 |
| 12 | 10 | 1053 | Example 4 |
| 13 | 10 | 1140 | Example 4 |
| 14 | 1 | 450 | Comparative Example 1 |
| 15 | 10 | 600 | Comparative Example 1 |
| 16 | 50 | 700 | Comparative Example 1 |
| 17 | 1 | 720 | Comparative Example 2 |
| 18 | 10 | 1005 | Comparative Example 2 |
| 19 | 50 | 1100 | Comparative Example 2 |

It could be seen that yield of the methylated library of samples 1 to 13 was significantly higher than that of samples 14 to 19, indicating that the method for constructing the free DNA methylated library provided by the embodiment of the present disclosure could improve yield of the methylated library. The yield of the library could reflect the ligation efficiency of the adapter and the template DNA, and only the ligation product of the adapter and the template DNA could be amplified and enriched to form a library. Therefore, the ligation efficiency of the adapter of the method for constructing the free DNA methylation library of the Examples of the present disclosure was significantly higher than that of the method for constructing library of the Comparative Examples.

Moreover, the yields of the methylation libraries of samples 1 to 9 were similar, indicating that the three types of adapter sequences of Example 3 could achieve better ligation efficiency of adapter as the adapter sequences of Example 1.

**Table 12**

| Number of sample | Mean methylation rate of read bases 1-100 of 5'-3' Read2 (theoretical value 50%) | Mean methylation rate of read bases 100-150 of 5'-3' Read2 (theoretical value 50%) | note |
|---|---|---|---|
| 1 | 48.0% | 49.0% | Example 1 |
| 2 | 52.0% | 52.0% | Example 1 |
| 3 | 53.0% | 48.0% | Example 1 |
| 4 | 49.0% | 47.0% | Example 2 |
| 5 | 53.0% | 51.0% | Example 2 |
| 6 | 54.0% | 52.0% | Example 2 |
| 7 | 48.8% | 49.4% | Example 3 |
| 8 | 52.3% | 48.8% | Example 3 |
| 9 | 51.5% | 50.9% | Example 3 |
| 10 | 50.8% | 35% | Example 4 |
| 11 | 49.5% | 48% | Example 4 |
| 12 | 48.5% | 49% | Example 4 |
| 13 | 51.5% | 29% | Example 4 |
| 14 | 48.5% | 49.0% | Comparative Example 1 |
| 15 | 53.3% | 47.8% | Comparative Example 1 |
| 16 | 51.9% | 51.0% | Comparative Example 1 |
| 17 | 47.7% | 19.8% | Comparative Example 2 |
| 18 | 49.0% | 22.5% | Comparative Example 2 |
| 19 | 55.0% | 25.0% | Comparative Example 2 |

It could be seen that the methylation rates of different fragments of samples 1 to 9, 11, and 12 were relatively close to the theoretical value of 50%, indicating that the method for constructing the free DNA methylation library provided by the embodiment of the present disclosure could maintain the original methylation status of free DNA and ensure the accuracy of methylation rate detection. Moreover, when a molar ratio of dNTP to dUTP in the extension reaction was 0.5: 1 to 2: 1, the accuracy of methylation rate detection was higher.

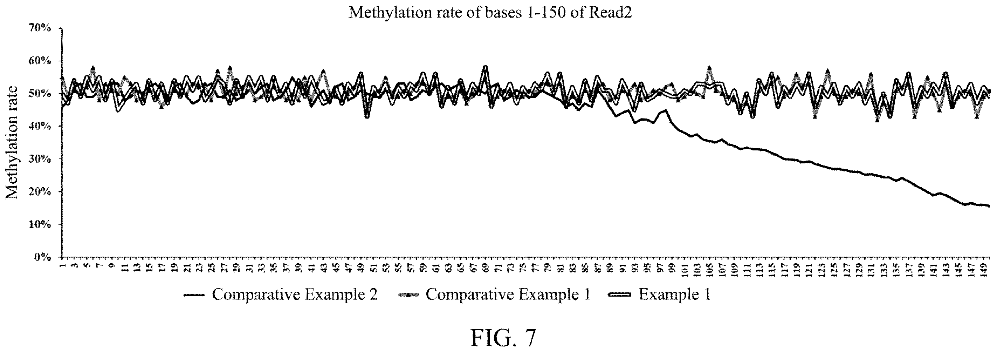

FIG. 7 was a comparison diagram of methylation rate results of bases 1-150 of Read2 sequence (5'-3' direction) in library sequencing according to Example 1 and Comparative Examples 1-2 of the present disclosure. The abscissa was the base reading position, and the ordinate was the base methylation rate at the position. It could be seen that the methylation rate at position 80-150 in Read2 of the library of Comparative Example 2 of the present disclosure was significantly lower than the theoretical value of 50%, and there was a problem that the methylation rate was reduced, while the methylation rate of bases 1-150 of library of Example 1 was close to the theoretical value of 50%, which effectively solved the problem that the methylation detection of free DNA was reduced at ends, and improved the accuracy of the methylation rate detection.

### Example 5

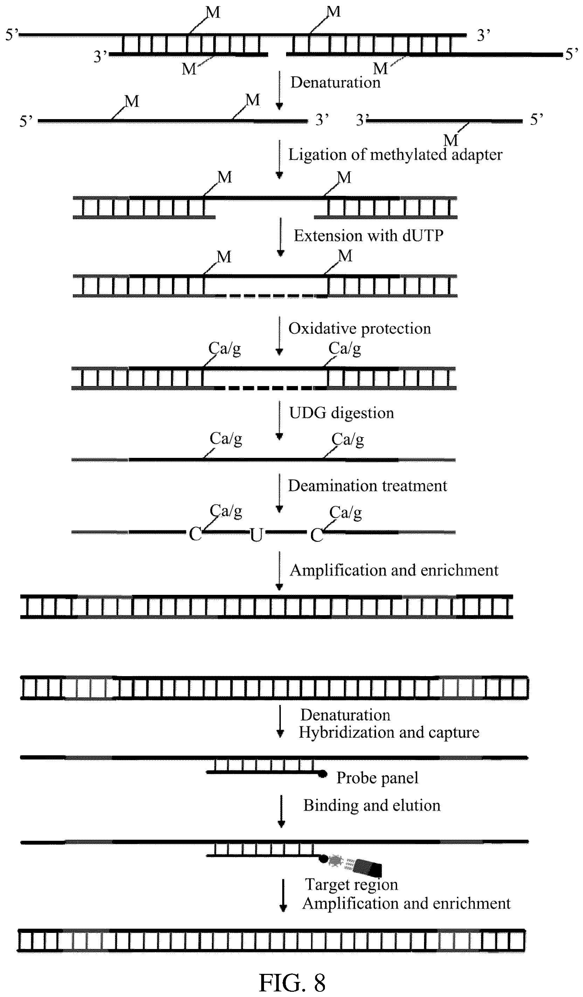

The present example provided a method for DNA hybridization and capture, which was performed employing the operation flow shown in FIG. 8.

The probe Panel (Cat. No.: 105520), Blocker Solution (Cat. No.: 100578) and Enhancer (Cat. No.: 100983) employed were all purchased from Twist Bioscience;

Cot-1 DNA (cat. no. 15279101) was purchased from Thermo Fisher, and Kapa Hifi hotstart ready Mix (cat. no. kk2601) from Roche;

Other raw materials and reagents were common commercial products.

The method for DNA hybridization and capture of the present example included:
(1) constructing a permethylated library using the method of Example 1 described above, labeling as libraries 1, 2, and 3;
(2) preparation of hybridization reagent: preparing hybridization solution according to Table 13, labeling as Hyb-1, fully mixing after preparation, and ready for use;

**Table 13**

| Reagent name | Volume (µL) | Final concentration |
|---|---|---|
| 10× SSPE | 100 | 1× |
| 10× Denhardt's | 100 | 1× |
| 100 mM ethylenediamine tetraacetic acid | 100 | 10 mmol/L |
| 2% Sodium Dodecyl Sulfonate | 100 | 0.20% |
| 1 % Tween 80 | 100 | 0.10% |
| 50% dimethyl sulfoxide | 10 | 5% |
| Betaine (10 mol/L) | 100 | 1 mol/L |
| Single strand binding protein SSB (500 ng/uL) | 50 | 25 ng/uL |
| DNase/RNase-Free Water (enzyme-free sterile water) | 340 | / |

Herein, EDTA contained in SSPE was at a final concentration of 10 mmol, and additional EDTA should be added to obtain a final concentration of 20 mmol.

In the sequential steps, the methylated library needed to undergo high-temperature denaturation, and then a single-stranded DNA molecule hybridized with a probe, for example, at a hybridization temperature of 60 °C. If the single stranded DNA molecule was renatured, the hybridization efficiency of the probe would be affected. In order to solve this problem, the example of the present disclosure employed single stranded binding protein SSB to bind a single-stranded DNA molecule, and a tetramer formed by the SSB specifically binds 8-16 bases, avoiding a renaturation of the single-stranded DNA, and effectively improving the hybridization efficiency.

(3) Hybridization reaction: 187.5 ng libraries 1, 2, 3 were respectively taken in a 1.5 mL centrifuge tube, fully mixed and centrifuged, labeled as sample 20, then the probes Panel, Cot-1 DNA, and Blocker Solution were thawed and mixed upside down, and a reagent system as shown in Table 14 was prepared in a sterilized PCR tube:

**Table 14**

| Components | Volume (uL) |
|---|---|
| Methylation libraries (1, 2, 3) | 187.5 ng |
| Probe Panel | 4 |
| Blocker Solution | 10 |

Cot-1 DNA was a kind of placental DNA, with a size mainly in a range of 50 to 300 bp, and rich in repeated DNA sequences, which could effectively block the repeated DNA sequences in the target region and reduce non-specific hybridization.

The reagent system in the sterilized PCR tube was fully mixed and briefly centrifuged, the sterilized PCR tube was placed in a vacuum concentration centrifuge, concentrated to dry powder, and then a reagent system as shown in Table 15 was prepared in the centrifuge tube:

**Table 15**

| Components | Volume (µL) |
|---|---|
| Hyb-1 | 20 |
| Enhancer | 20 |
| Total | 40 |

Gently blowing and fully mixing, centrifuging briefly, putting into the PCR instrument for hybridization reaction, the conditions of hybridization reaction was as shown in Table 16;

**Table 16**

| Temperature | Time |
|---|---|
| Denaturation temperature at 95 °C | 5 min |
| Hybridization temperature at 60 °C | 16 h |

### (4) Streptavidin magnetic beads cleaning:

1) 40 min before the completion of the reaction of the previous step, removing the streptavidin magnetic beads from 4 °C and equilibrating at room temperature for 30 min;
2) pipetting 100 µL beads into a 1.5. mL low-adsorption centrifuge tube for bead cleaning using Beads Binding buffer;
3) adding 200 µL Bead Binding Buffer to the centrifuge tube, gently blowing and sucking 10 times to mix, instantaneously centrifuging, placing on a magnetic rack for a few minutes, waiting for the liquid to be completely clarified, and removing the supernatant with a pipette, removing centrifuge tube from the magnetic rack;
4) repeating step 3) twice;
5) adding 200µL magnetic bead suspension into the centrifuge tube, gently blowing to mix, and transferring all magnetic bead suspension to a new 1.5 mL Low adsorption PCR tube;

(5) eluent preparation: preparing eluents WB1 and WB2 according to tables 17 and 18 below.

**Table 17**

| WB1 component | Volume (µL) | Final concentration |
|---|---|---|
| 10× SSPE | 400 | 1× |
| Tetramethyl ammonium chloride solution (4 M) | 1000 | 1M |
| 20% formamide | 1000 | 5.00% |
| 2% SDS | 200 | 0.10% |
| DNase/RNase-Free Water (enzyme-free sterile water) | 1400 | / |

**Table 18**

| WB2 component | Volume (µL) | Final concentration |
|---|---|---|
| 10× SSPE | 400 | 1× |
| 2% SDS | 100 | 0.05% |
| DNase/RNase-Free Water (enzyme-free sterile water) | 3500 | / |

Since the ratio of A and T bases of the DNA strands in the methylated library increased after methylation transformation, tetramethyl ammonium chloride in the eluent could increase a melting temperature of DNA strands rich in A and T bases and increase the TM value of a region rich in A and T bases in the DNA strand, making it close to the TM value of the DNA strand with a normal ratio of A and T bases. Test results of gradient concentrations of tetramethyl ammonium chloride (0.1 M, 0.5 M, 1M, 1.5 M, 2M) showed that the concentration of tetramethyl ammonium chloride was better at 1M. In the eluting step, the eluent was incubated to 48 °C, which could effectively wash away the non-specific hybridization products, and ensure that the specific hybridization products rich in A and T bases remained.

Formamide could reduce the TM value of the DNA strand, and every increase of 1% formamide could reduce the TM value of about 0.7 °C. After incubation to 48 °C, the elute containing 5% formamide could enhance the specificity of hybridization to a greater extent, and reduce the loss of hybridization products in the target region to a lower extent.
(6) Elution of hybridization products: fully mixing 200 µL magnetic bead suspension and hybridization products, incubateding at room temperature for 30min, then washing with Eluent WB1 at 65 °C and incubated at 65 °C for 5min, repeated 3 times; finally, washed with Eluent WB2, and incubated at 48 °C for 5min and repeat for 3 times.
(7) Amplification of elution products: thawing the amplification primers (Amplification Primers, cat. No. 100983, purchased from Twist Bioscience) and KAPA HiFi Hotstart Ready Mix (kk2601) and mixing upside down, and preparing a reagent system as shown in Table 19 in a sterilized PCR tube:

**Table 19**

| Components | Volume (µL) |
|---|---|
| Elution product of step (6) | 22.5 |
| KAPA HiFi Hotstart Ready Mix | 25 |
| Amplification Primer | 2.5 |
| Total | 50 |

Gently blowing to mix, and centrifuging briefly, putting into the PCR instrument for the following amplification reaction. The reaction conditions of the amplification reaction were shown in Table 20.

**Table 20**

| Temperature | Time | Number of cycles |
|---|---|---|
| 98 °C | 45 s | 1 |
| 98 °C | 15 s | 14 |
| 60 °C | 30 s | |
| 72 °C | 30 s | |
| 72 °C | 1 min | 1 |
| 4 °C | Holding | |

(8) Purification of product: adding 90 µL of magnetic beads for purification .to the product of step (7), fully mixing, standing at room temperature for 5 min, placed on a magnetic rack for about 5 min to allow the magnetic beads being completely adsorbed and the solution being clarified, carefully removing the supernatant; adding 200 µL of freshly formulated 80 volume % ethanol for rinsing, incubating at room temperature for 30 s to 60 s, removing the supernatant carefully, repeating once; after the magnetic beads were dried, adding 32 µL of ultrapure water to elute, leaving at room temperature for 3 min and placing on a magnetic rack, after the solution was clarified, pipetting 30 µL of supernatant for use.
(9) On-machine sequencing: diluting the methylated library obtained in step (8) to 1 ng/µL, taking out 1 µL for detection with Agilent 4200 Tapestation system (Agilent, USA); in addition, taking out 1 µL more for qPCR detection, and determining a concentration on the machine according to the detection results. According to the concentration obtained in the previous step, diluting the library to requirement on the instrument (2nmol), and performing PE150 sequencing on Novaseq sequencing platform of Illumina Company, with the data volume of 10 G for each sample.

### Comparative Example 3

The sample employed in this Comparative Example was selected from the methylation libraries 1, 2, and 3 employed in Example 5. 187.5 ng of each of libraries 1, 2, and 3 were taken in a 1.5 mL centrifuge tube, fully mixed and centrifuged, and labeled as sample 21. The subsequent experiment flow for hybridization and capture was purchased from Twist Bioscience hybridization capture reagents, which were different from Example 5 in that the hybridization reaction step used Fast Hybridization Reagent provided by the kit, and the hybridization product elution step used Washing Buffer 1 and Washing Buffer 2 provided by the kit.

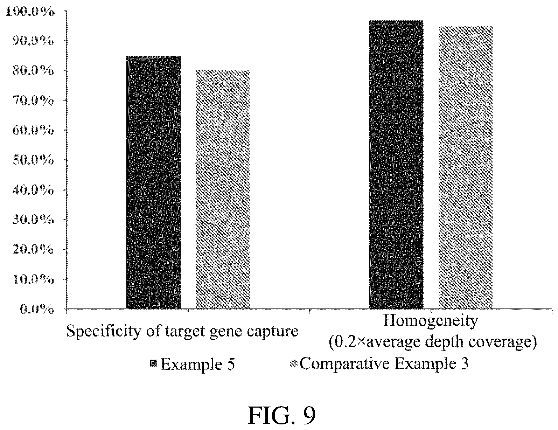

FIG. 9 was a comparison diagram of specificity and homogeneity of target gene capture in the methods for capture according to Example 5 and Comparative Example 3 of the present disclosure.

It could be seen that by introducing betaine, dimethyl sulfoxide, and single strand binding protein into the hybridization reagent in Example 5 of the present disclosure, the hybridization efficiency was improved, and the capture homogeneity was improved; in the elution reaction, the eluent WB1 was added with tetramethyl ammonium chloride and formamide, which enhanced the capture specificity of the final target gene.

Although the implementations of the present disclosure are disclosed above, the contents are only implementations used for ease of understanding of the present disclosure, but not intended to limit the present disclosure. Those skilled in the art may make any modification and change in the forms and details of the implementations without departing from the essence and scope of the present disclosure. However, the scope of protection of the present disclosure should still be subject to the scope defined by the attached claims.

## Claims

1. A method for constructing a DNA methylation library, comprising:
providing a test sample containing a plurality of double-stranded DNA fragments;
denaturing the double-stranded DNA fragments into single-stranded DNA;
performing a ligation reaction on the single-stranded DNA and a double-stranded adapter sequence to obtain a single-stranded DNA connected with the double-stranded adapter sequence;
performing an extension reaction on the single-stranded DNA connected with the double-stranded adapter sequence, the extension reaction forming an extended fragment, the extended fragment being connected to the double-stranded adapter sequence of the single-stranded DNA connected with the double-stranded adapter sequence to form a double-stranded DNA connected with the double-stranded adapter sequence;
performing an oxidation reaction on cytosine carrying methylation modification in the double-stranded DNA connected with the double-stranded adapter sequence to form a protected cytosine and obtain a double-stranded DNA carrying the protected cytosine;
removing the extended fragment from the double-stranded DNA carrying the protected cytosine to obtain a single-stranded DNA carrying the protected cytosine;
performing a deamination reaction on an unmethylated cytosine in the single-stranded DNA carrying the protected cytosine to form an uracil and obtain a single-stranded DNA carrying the uracil; and
performing an amplification reaction on the single-stranded DNA carrying the uracil to obtain a DNA methylation library.

2. The method for constructing of claim 1, wherein the single-stranded DNA is modified prior to ligation with the double-stranded adapter sequence, which modification doesn't comprise end repair, and/or end A tailing; and/or,
the modification comprises phosphorylation.

3. The method for constructing of claim 1, wherein the extended fragment comprises at least one uracil, and the extension reaction comprises realizing the extension reaction with an extension reagent including dNTP and dUTP, wherein the dNTP doesn't comprise dTTP.

4. The method for constructing of claim 3, wherein a molar ratio of dNTP to dUTP is 0.5:1 to 2:1; and
a reaction condition of the extension reaction comprises: a reaction temperature of 20 °C, and a reaction time of 15 min; a reactor is maintained at a temperature of 4 °C after the extension reaction is completed.

5. The method for constructing of claim 3, wherein removing the extended fragment from the double-stranded DNA carrying the protected cytosine comprises:
performing a digestion reaction employing an UDG enzyme to degrade the uracil in the extended fragment, thereby removing the extended fragment from the double-stranded DNA carrying the protected cytosine;
wherein, the digestion reaction comprises a first digestion stage and a second digestion stage; the first digestion stage is at a reaction temperature of 37 °C, and for a reaction time of 20 min; the second digestion stage is at a reaction temperature of 50 °C, and for a reaction time of 5 min.

6. The method for constructing of claim 1, wherein performing a ligation reaction on the single-stranded DNA and a double-stranded adapter sequence, and performing an extension reaction on the single-stranded DNA connected with the double-stranded adapter sequence to form a double-stranded DNA connected with the double-stranded adapter sequence, comprises:
connecting double-stranded adapter sequences to both ends of single-stranded DNA respectively to amplify and generate double-stranded DNA containing paired-end adapters; and/or
connecting a first double-stranded adapter sequence to a first end of the single-stranded DNA to amplify and generate a double-stranded DNA containing a single-ended adapter, and connecting a second double-stranded adapter sequence to a second end of the single-stranded DNA to generate a double-stranded DNA containing a paired-end adapter.

7. The method for constructing of any one of claims 1 to 6, wherein the double-stranded adapter sequence comprises cytosines, and all of the cytosines are methylated.

8. The method for constructing of any one of claims 1 to 7, wherein the double-stranded adapter sequence comprises a first double-stranded adapter sequence which is a splint sequence formed by a first oligonucleotide sequence and a second oligonucleotide sequence, and a second double-stranded adapter sequence which is a splint sequence formed by a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than that of the third oligonucleotide sequence; and
overhangs of the second oligonucleotide sequence and the fourth oligonucleotide sequence can be complementary to and paired with the single-stranded DNA.

9. The method for constructing of any one of claims 1 to 8, wherein the double-stranded adapter sequence comprises a label nucleotide, wherein, the label nucleotide comprises one or more of molecular barcode sequence, unique molecular identifier, sample-specific index sequence, universal primer site, and sequencing oligonucleotide for clustering and/or sequencing; and/or
partial sequences of the above label nucleotide.

10. The method for constructing of claim 9, wherein, the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4.

11. The method for constructing of claim 9, wherein, the first oligonucleotide sequence, the second oligonucleotide sequence, the third oligonucleotide sequence and the fourth oligonucleotide sequence carry or do not carry, at the ends thereof, a modification group selected from any one or more of the phosphate group Pho, amino-substituted methylene, dideoxycytosine nucleoside and C3/C6 Spacer; wherein, the methylene in the amino-substituted methylene has 6 to 12 carbon atoms, and C3, C6 in the C3/C6 Spacer represents that the methylene in the end scaffold of the Spacer and the oligonucleotide sequence has 3 or 6 carbon atoms.

12. The method for constructing of any one of claims 1 to 7, further comprising a purification process comprising any one or more of:
after the extension reaction and before the oxidation reaction, capturing the product obtained by the extension reaction with magnetic beads, and washing the product of the extension reaction captured by the magnetic beads with ethanol and water;
after removing the extended fragment from the double-stranded DNA carrying the protected cytosine and before the deamination reaction, capturing the product obtained after removing the extended fragment with magnetic beads, and washing the product obtained after removing the extended fragment and captured by the magnetic beads with ethanol and water; and
after the deamination reaction and before the amplification reaction, capturing the product obtained by the deamination reaction with magnetic beads, and washing the product of the deamination reaction captured by the magnetic beads with ethanol and water.

13. The method for constructing of any one of claims 1 to 12, further comprising:
hybridization reaction;
eluting and amplifying the products of the hybridization reaction; and
sequencing the amplified products.

14. The method for constructing of claim 13, wherein the hybridization reaction comprises a hybridization reaction employing a hybridization reagent comprising betaine, dimethyl sulfoxide, and single strand binding protein.

15. The method for constructing of claim 13, wherein the eluting comprises adding a boosting agent comprising tetramethyl ammonium chloride and formamide.

16. The method for constructing of claim 15, wherein the tetramethyl ammonium chloride is at a concentration of from 0.5 M to 1.5 M.

17. The method for constructing of any one of claims 1 to 16, wherein the double-stranded DNA fragment is a cell-free DNA fragment and/or a genomic DNA fragment.

18. The method for constructing of any one of claims 1 to 16, wherein the test sample is from whole blood, blood components, plasma, serum, urine, stool, saliva, tissue biopsy, pleural fluid, pericardial fluid, cerebrospinal fluid, or peritoneal fluid.

19. The method for constructing of any one of claim 1 to 16, comprising being used in combination with other omics.

20. Use of the method for constructing of any one of claims 1-19 in sequencing, detecting the presence or absence of cancer, determining cancer status, monitoring cancer progression, and/or determining cancer classification.

21. A free DNA methylation library obtained by the method for constructing the DNA methylation library of any one of claims 1 to 19.

22. A method for DNA hybridization and capture comprising:
hybridization reaction;
eluting and amplifying the products of the hybridization reaction; and
sequencing the amplified products.

23. The method for DNA hybridization and capture of claim 22, wherein the hybridization reaction comprises a hybridization reaction employing a hybridization reagent comprising betaine, dimethyl sulfoxide, and single strand binding protein.

24. The method for DNA hybridization and capture of claim 22 or 23, wherein the eluting comprises adding a boosting agent comprising tetramethyl ammonium chloride and formamide.

25. The method for DNA hybridization and capture of claim 24, wherein the tetramethyl ammonium chloride is at a concentration of from 0.5 M to 1.5 M.

26. A kit for constructing a methylation library, for use in performing the method for constructing the DNA methylation library of any one of claims 1 to 19.

27. The kit of claim 26, wherein the double-stranded adapter sequence comprises a first double-stranded adapter sequence which is a splint sequence formed by a first oligonucleotide sequence and a second oligonucleotide sequence, and a second double-stranded adapter sequence which is a splint sequence formed by a third oligonucleotide sequence and a fourth oligonucleotide sequence;
wherein, the first oligonucleotide sequence is complementary to and paired with at least a portion of the second oligonucleotide sequence, and the third oligonucleotide sequence is complementary to and paired with at least a portion of the fourth oligonucleotide sequence;
the second oligonucleotide sequence is at a length greater than that of the first oligonucleotide sequence, and the fourth oligonucleotide sequence is at a length greater than that of the third oligonucleotide sequence;
overhangs of the second oligonucleotide sequence and the fourth oligonucleotide sequence can be complementary to and paired with the single-stranded DNA; and/or
the first oligonucleotide sequence is a sequence having at least 70% identity to the sequence set forth in SEQ ID NO. 1, the second oligonucleotide sequence is a sequence having at least 70% identity to the sequence set forth in SEQ ID NO. 2, the third oligonucleotide sequence is a sequence having at least 70% identity to the sequence set forth in SEQ ID NO. 3, and the fourth oligonucleotide sequence is a sequence having at least 70% identity to the sequence set forth in SEQ ID NO. 4; and/or
the kit further comprises an extension reagent used for the extension reaction, the extension reagent comprises: dNTP and dUTP, wherein the dNTP doesn't comprise dTTP, and a molar ratio of the dNTP to dUTP is 0.5:1 to 2:1.

28. A kit for hybridization and capture, for use in performing the method for DNA hybridization and capture of any one of claims 22 to 25.

29. The kit for hybridization and capture of claim 28, comprising: a hybridization reagent employed for the hybridization reaction and a boosting agent employed for the eluting;
wherein the hybridization reagent comprises betaine, dimethyl sulfoxide and single strand binding protein; and/or,
the eluting comprises adding a boosting agent comprising tetramethyl ammonium chloride and formamide; and
optionally, the tetramethyl ammonium chloride is at a concentration of from 0.5 M to 1.5 M.
